# EUROPÄISCHE PATENTSCHRIFT

(11) **EP 3 253 801 B1**
(45) Veröffentlichungstag und Bekanntmachung des Hinweises auf die Patenterteilung: **04.03.2020**
(21) Anmeldenummer: 16702408.2
(22) Anmeldetag: 01.02.2016
(51) Int. Cl.: C07K 16/40, C07K 16/30, A61P 35/00, A61K 39/00

(54) **EINZELDOMÄNEN-VHH-FRAGMENTE, DIE AN KATALASE BZW. SUPEROXIDDISMUTASE BINDEN UND DIESE INHIBIEREN ZUR BEHANDLUNG VON TUMORERKRANKUNGEN**
SINGLE DOMAIN-VHH FRAGMENTS BIND TO AND INHIBIT CATALASE, RESPECTIVELY SUPEROXIDE DISMUTASE, FOR USE IN THE THERAPY OF TUMORS
FRAGMENT DE VHH A DOMAINE INDIVIDUEL, SE LIANT À ET INHIBANT LA CATALASE, RESPECTIVEMENT LA SUPEROXYDE DISMUTASE, POUR UTILISATION DANS LE TRAITEMENT DES TUMEURS

(30) Priorität: 02.02.2015 EP 15153421
(43) Veröffentlichungstag der Anmeldung: 13.12.2017
(73) Patentinhaber: Bauer, Georg, 79104 Freiburg (DE); Motz, Manfred, 80689 München (DE)
(72) Erfinder: Bauer, Georg, 79104 Freiburg (DE); Motz, Manfred, 80689 München (DE)
(74) Vertreter: Keller, Günter
(86) Internationale Anmeldenummer: PCT/EP2016/052016
(87) Internationale Veröffentlichungsnummer: WO 2016/124512

(56) Entgegenhaltungen:
- EP-A1- 2 535 352
- EP-A1- 2 679 225
- WO-A1-2014/191493
- KIJANKA M. ET AL.: "Nanobody-based cancer therapy of solid tumors.", NANOMEDICINE, vol. 10, no. 1, January 2015 (2015-01), pages 161-174,
- BAUER G. & MOTZ M.: "The antitumor effect of single-domain antibodies directed towards membrane-associated catalase and superoxide dismutase.", ANTICANCER RES., vol. 36, November 2016 (2016-11), pages 5945-5956,

## Beschreibung

Die vorliegende Erfindung beruht auf dem unerwarteten Befund, dass Antigen bindende Konstrukte, insbesondere Single-Domain-VHH-Fragmente (Nanobodies), die entweder humane Katalase oder humane SOD1 neutralisieren, eine mehr als hundertfach größere Effizienz bei der zur Tumorapoptose führenden Reaktivierung interzellulärer, ROSgetriebener Signalwege herbeiführen als die aus einem leichten und schweren Kettenanteil bestehenden klassischen rekombinant hergestellten Fab-Fragmente oder monoklonalen Antikörper, die beide ebenfalls neutralisierende Wirkung auf Katalase bzw. SOD1 zeigen. Durch die Applikation von Single-Domain-VHH-Fragmenten gegen Katalase oder SOD1 wird das interzelluläre ROS-Signaling reaktiviert und die Zellen in Apoptose getrieben. Single-Domain-VHH-Fragmente gegen eines der beiden protektiven Enzyme sind ausreichend, um den Schutz der Tumorzellen vor ROS-Signaling aufzuheben, obwohl die Schutzwirkung von Katalase und SOD teilweise redundant und miteinander verschränkt ist. Dies beruht darauf, dass die SOD nur eine partiell protektive Wirkung erreicht, aber die Hemmung der SOD zu einer indirekten Hemmung der Katalase durch die nun in höherer Konzentration vorliegenden Superoxidanionen führt. Die Qualität der durch Hemmung von SOD oder Katalase bewirkten Signalwege ist unterschiedlich. Hemmung der Katalase erlaubt die sequenzielle Aktivierung des NO/Peroxynitrit- und des HOCI-Weges, während die Hemmung der SOD ausschließlich zur Reaktivierung des NO/Peroxynitritweges führt. Von hohem therapeutischem Wert ist der Befund, dass Single-Domain-VHH-Fragmente gegen SOD und Katalase synergistisch kooperieren und dass sich dieser Synergieeffekt in einer bevorzugten Ausführungsform in einem Hybridmolekül bündeln lässt. Auf dieser Basis wird eine vorteilhafte Form der Therapie von Tumoren mit Antigen bindenden Konstrukten, die an SOD oder an Katalase oder an beide Antigene binden, offenbart.

### Hintergrund der vorliegenden Erfindung

Es wurde erst vor wenigen Jahren erkannt, dass Reaktive Sauerstoff- und Stickstoffspezies (zusammenfassend abgekürzt als reactive oxygen species = "ROS") neben ihrer ungerichteten mutagenen Wirkung auch spezifische Signalfunktionen ausführen können (Bauer et al., Chimica, 62, 1-9, 2008). Die Wahrnehmung dieses Befundes wurde vor allem auch dadurch erschwert, dass durch bestimmte ROS sehr häufig gegenläufige biologische Effekte hervorgerufen werden können.

Die EP 11170076.1 offenbart, dass die Hemmung der protektiven membranständigen Katalase von Tumorzellen zu einer Reaktivierung des interzellulären ROS-Signaling und damit zum selektiven Zelltod der Tumorzellen führt. Eine Hemmung der protektiven Katalase kann auch auf indirektem Weg durch Hemmung einer membranständigen SOD erreicht werden, da nach Hemmung der SOD die von der benachbarten, ebenfalls membranständigen NADPH-Oxidase generierten Superoxidanionen nicht mehr in H₂O₂ umgewandelt werden. Dadurch liegt nun eine ausreichend hohe lokale Konzentration freier Superoxidanionen vor, die über einen Ein-Elektronen-Transfer das aktive Intermediat Compound I der Katalase (CATFe^{IV}=O·⁺) in das inaktive Intermediat Compound II (CATFe^{IV}=O) überführen, und außerdem aktive Katalase (CATFe^{III}) in das inaktive Compound III (CATFe^{III}O₂·⁻) umwandeln. Durch diese beiden Reaktionen wird die Aktivität der Katalase wirkungsvoll gehemmt (Kono Y and Fridovich I: Superoxide radical inhibits catalase. J Biol Chem 257:5751-5754, 1982. Shimizu N, Kobayashi K and Hayashi K: The reaction of superoxide radical with catalase. Mechanism of the inhibition of catalase by superoxide radical. J Biol Chem 259: 4414-4418, 1984).

Für die Rolle von ROS bei der Mehrstufenonkogenese kann heute allerdings ein einigermaßen klares Bild gezeichnet werden, dessen Kenntnis wegweisend für die Etablierung neuer selektiver Tumortherapieverfahren sein könnte (zusammengefasst in Bauer G. Tumor cell protective catalase as a novel target for rational therapeutic approaches based on specific intercellular ROS signaling. Anticancer Res. 32: 2599-2624, 2012; Bauer G. Targeting extracellular ROS signaling of tumor cells. Anticancer Res. 34: 1467-1482, 2014; Bauer G. Increasing the endogenous NO level causes catalase inactivation and reactivation of intercellular apoptosis signaling specifically in tumor cells. Redox Biol. 6: 353-371. 2015):
1) Die mutagene Wirkung von ROS trägt zum klassischen Schritt der "Tumorinitiation" bei.
2) ROS sind für den Schritt der "Tumorpromotion" von entscheidender Bedeutung, ohne dass der zugrunde liegende Mechanismus im Detail geklärt ist. So induzieren die meisten der bekannten Tumorpromotoren erhöhte ROS-Spiegel und das spezifische Wegfangen von ROS hemmt die Wirkung der meisten Tumorpromotoren.
3) Die maligne Transformation von Zellen führt regelmäßig zur Expression einer membranständigen NADPH-Oxidase (NOX1). Die kontinuierliche Aktivität dieses Enzyms ist sowohl für die Proliferation der malignen Zellen, als auch für die Aufrechterhaltung des transformierten Zustandes von essentieller Bedeutung. NOX1 generiert extrazelluläre Superoxidanionen, deren Dismutationsprodukt H₂O₂ einen autokrinen Proliferationsstimulator für die Zellen darstellt.
4) Das Generieren extrazellulärer Superoxidanionen stellt jedoch auch die Grundlage für die Entstehung von interzellulären ROS-gesteuerten Signalwegen dar, die selektiv Apoptose der transformierten Zellen induzieren. Von besonderer Bedeutung sind dabei der "HOCI-Signalweg" und der "NO/Peroxynitritsignalweg", die weiter unten im Detail erläutert werden.
   Die spezifische ROS-abhängige Apoptoseinduktion in transformierten Zellen wird daher als potenzieller Eliminationsmechanismus für maligne Zellen diskutiert.
5) Erfolgreiche Tumorentstehung setzt voraus, dass sich ROS-produzierende maligne Zellen vor der destruktiven Wirkung der ROS-Signalwege schützen, ohne dabei die für ihre Proliferation notwendige autokrine ROS-Produktion zu beeinträchtigen. Daraus resultiert der regelmäßig und in allen bisher untersuchten Tumorsystemen gefundene Phänotyp von Tumorzellen, der durch konstitutive NOX1-Aktivität und parallele Aktivität einer membranständigen Katalase gekennzeichnet ist. Zusätzlich findet eine weitere Modulation des ROS-Signaling durch membranständige Superoxid-Dismutase (SOD) statt.
6) Die gezielte Hemmung dieser membranständigen protektiven Enzyme ermöglicht eine ROS-abhängige selektive Apoptoseinduktion in Tumorzellen und besitzt daher ein großes therapeutisches Potenzial.

Unter Berücksichtigung der oben kurz skizzierten und nachfolgend weiter erläuterten, im allgemeinen aufgrund der Wechselwirkungen äußerst komplexen und schwer vorhersagbaren Mechanismen offenbart die vorliegende Erfindung Antigen bindende Konstrukte, im weiten Sinne, die insbesondere für die Tumortherapie verwendet werden können.

Die EP 2 679 225 offenbart die Verwendung von anti-SOD und anti-Katalase Antikörpern in der Krebsbehandlung.

Die WO 2014/191493 offenbart Single-Domain-Antibodies gegen SOD1 und deren Verwendung bei der Behandlung von ALS (Amylotrophe Laterale Sklerose). Dabei soll der Spiegel an pathogener SOD1 bei Patienten mit ALS durch gegen SOD1 gerichtete Single-Domain-Antibodies verringert werden, um eine positive Wirkung zu erreichen.

### Beschreibung der Erfindung

Gegenstand der vorliegenden Erfindung sind daher Antigen bindende Konstrukte, nämlich Single-Domain-VHH-Fragmente, auch Nanobodies genannt, die spezifisch entweder an die Superoxiddismutase oder an die Katalase oder an beide Enzyme, nämlich Superoxiddismutase und Katalase binden und diese inhibieren gemäß den Patentansprüchen.

Die erfindungsgemäßen Antigen bindenden Konstrukte sind Single-Domain-VHH-Fragmente gegen Katalase oder SOD. Hierbei wird mit weniger als einem Prozent der Konzentration vergleichbarer konventioneller Fab-Fragmente der gleiche Apoptose-auslösende Effekt erreicht, obwohl aufgrund des unterschiedlichen Aufbaus der VHH- und der Fab-Fragmente (eine versus zwei Ketten) nur ein Konzentrationsunterschied von 50 % zu erwarten gewesen wäre. Dieser dramatische Unterschied in der Wirksamkeit wurde für alle Katalase oder SOD neutralisierenden Single-Domain-VHH-Fragmente gefunden und stellt somit deren charakteristisches Merkmal dar. Dieses weist darauf hin, dass es zwischen der Kinetik der Bindung und der Reversibilität der Bindung für konventionelle Fab-Fragmente und Single-Domain-VHH-Fragmente einen bisher nicht erkannten und auch nicht vorhersehbaren grundsätzlichen Unterschied mit weitreichenden Konsequenzen für die Wirkung der Fragmente, und damit auch für deren sehr deutlich verbesserte therapeutische Einsatzmöglichkeiten geben muss. Als Grundlage zur Erklärung dieser Entdeckung kann der Unterschied zwischen der Erkennung von Epitopen durch klassische Fab-Fragmente (bestehend aus Antigen-bindenden Anteilen der schweren und der leichten Kette des Immunglobulinmoleküls) und durch VHH-Fragmente der Single-Domain-Antikörper (die nur aus dem Antigen-bindenden Anteil schwerer Ketten bestehen) herangezogen werden. Während klassische Fab-Fragmente eine durch ihre räumliche Struktur und ihre Oberflächenladung definierte Bindung an ein spezifisches Epitop eingehen und dieses gewissermaßen umfassen, weisen Single-Domain-VHH-Fragmente eine Pfropfen-förmige Struktur auf, die sich in eine bezüglich Form und Ladung komplementäre Vertiefung des Zielmoleküls einpasst (Muyldermans S. Nanobodies: Natural single-domain antibodies. Ann. Rev. Biochem. 82: 775-797, 2013). Es wird vermutet, dass die in dieser Anmeldung offenbarten Single-Domain-VHH-Fragmente, die entweder Katalase oder SOD wirkungsvoll hemmen, in den für diese Enzyme charakteristischen Trichter binden, der die Substratmoleküle zum aktiven Zentrum leitet. Es ist denkbar, dass nach einer solchen Bindung eine Konformationsänderung der Enzyme induziert wird, die die Rückreaktion, d.h. der Aufhebung der Bindung zwischen Single-Domain-VHH-Fragment und Antigen wirkungsvoll verhindert. Dadurch wird die biophysikalische Kenngröße "Affinität", die durch die Reaktionskonstanten der Hin- und Rückreaktion bestimmt ist, wesentlich erhöht, da die Rückreaktion wegfällt. In der Anwendungspraxis wird dadurch eine weitaus effizientere neutralisierende Wirkung durch Single-Domain-VHH-Fragmente erreicht, als dies konventionelle Fab-Fragmente erzielen können, selbst wenn deren Bindung mit gleicher Effizienz erfolgen sollte. Dieser essentielle Vorteil von Single-Domain-VHH-Fragmenten bei der Hemmung spezifischer Enzyme ist bisher nicht beschrieben worden und ist überraschend.

Es ist nicht auszuschließen, dass bestimmte Single-Domain-VHH-Fragmente ihre hemmende Wirkung auf Katalase oder SOD dadurch erreichen, dass ihre Bindung an eine vom aktiven Zentrum verschiedene Position im Enzym erfolgt, aber durch die bekannten allosterischen Fernwirkungen innerhalb eines Proteins dadurch eine hemmende Wirkung auf die Enzymaktivität erreicht wird. Aufgrund der erfindungsgemäßen Ergebnisse kann auch für diese Variante der Wirkungskette angenommen werden, dass die Affinität der Single-Domain-VHH-Fragmente für den die allosterische Hemmung auslösenden Bereich auf den Enzymen wesentlich höher sein muss als dies durch klassische Fab-Fragmente erreicht werden kann. Daher ist die Erfindung nicht auf Single-Domain-VHH-Fragmente beschränkt, die direkt am aktiven Zentrum der Enzyme binden.

Was die Struktur von künstlich hergestellten Antikörperfragmenten angeht, ist darauf hinzuweisen, dass es mittlerweile eine enorme Vielfalt von unterschiedlichen Strukturen gibt. In dem Artikel von Holliger et al. (2005), Nature Biotechnology, Vol. 23, Nr. 9, Seiten 1126-1136, sind verschiedenste Strukturen von Antigen bindenden Molekülen dargestellt und erläutert. Neben den erfindungsgemäß eingesetzten VHH-Single-Domain-Fragmenten gibt es auch viele andere Strukturen, wie Fab-Fragmente, Single-Chain-Fᵥ, Diabodies, Minibodies, Triabodies und andere.

Die erfindungsgemäß eingesetzten Single-Domain-Antibody-Fragmente sind von Antikörpermolekülen von kamelartigen Tieren (Kamel, Lama, Alpaka) abgeleitet. Diese Antikörper weisen in der natürlichen Erscheinungsform einen Fc-Teil (mit CH2 und CH3) auf, bestehen aber nur aus zwei schweren Ketten, die wiederum jeweils einen VHH-Teil mit den CDRs aufweisen. Leichte Ketten besitzen diese Antikörper der Kamelartigen nicht. Diese VHH-Teile werden als Single-Domain-VHH-Fragmente oder auch Nanobodies bezeichnet.

Die erfindungsgemäßen Single-Domain-VHH-Fragmente können entweder als solche verwendet werden oder sie können auch chemisch modifiziert werden, um bestimmte Ziele zu erreichen. Die erfindungsgemäßen Single-Domain-VHH-Fragmente können entweder durch kovalente chemische Bindung oder durch andere chemische Wechselwirkungen, wie ionische Wechselwirkungen oder van der Waals-Kräfte mit anderen Komponenten verbunden sein, die einen vorteilhaften Effekt aufweisen. Es kann sich hierbei um Konjugate mit biologisch aktiven Molekülen, wie Liganden und/der Rezeptoren handeln, die an bestimmte Zellen binden können. Ebenso ist es möglich, dass die erfindungsgemäßen Single-Domain-VHH-Fragmente mit bildgebenden Materialien, wie Radionucleiden, farberzeugenden Enzymen und ähnlichem gekoppelt werden. Die erfindungsgemäßen Moleküle können auch mit Molekülen verbunden sein, die zu einer längeren Verweildauer im Körper führen; dies kann beispielsweise durch Verbindung mit größeren Polymeren wie beispielsweise Polyethylenglykol bewirkt werden. Verschiedene mögliche Einsatzoptionen und Modifikationen der Single-Domain-VHH-Fragmente werden in dem Übersichtsartikel von Eyer et al., Veterinarni Medicina (2012), 9, Seiten 439-531, offenbart. Auf diese Literaturstelle wird ausdrücklich verwiesen.

Ein wesentlicher Aspekt der vorliegenden Erfindung ist, dass die Antigen bindenden Konstrukte nicht nur spezifisch an die jeweiligen Zielmoleküle (Superoxiddismutase bzw. Katalase) binden, sondern diese auch inhibieren. Die Testung dieses Aspekts erfolgt durch übliche, dem Fachmann wohlbekannte Methoden. In einem geeigneten Testmodell, beispielsweise Tumorzellen, die das gewünschte Zielmolekül (Katalase oder Superoxiddismutase) auf der Zelloberfläche exprimieren oder in einem Testsystem, zu dem gereinigte SOD oder Katalase zugegeben wurden, werden die Substrate für die jeweiligen Enzyme zugegeben oder von den Zellen produziert. Dann wird entweder der Verbrauch der Edukte oder die Generierung der Produkte durch geeignete Messmethoden gemessen. Dabei können auch Verfahren zum Einsatz kommen, bei denen ein biologischer Vorgang, wie zum Beispiel Induktion der ROS-abhängigen Apoptose von Tumorzellen als Messgröße eingesetzt wird. Voraussetzung für die Aussagekraft dieser in der Regel sehr sensitiven biologischen Verfahren ist, dass das durch das zu testende Enzym abgebaute Substrat in einem kontrollierbaren und quantitativen Zusammenhang mit dem gemessenen biologischen Effekt steht. Dies ist für SOD und Katalase in besonderer Weise der Fall. SOD setzt zum Beispiel die für das interzelluläre ROS-Signaling notwendigen Superoxidanionen in H₂O₂ um und verhindert dadurch die für die HOCI-abhängige Apoptoseinduktion notwendige Interaktion zwischen Superoxidanionen und HOCI.

Daher kann die durch Single-Domain-VHH- oder Fab-Fragmente (Vergleich) vermittelte Hemmung der SOD dadurch bestimmt werden, dass die Apoptoseinduktion von Tumorzellen durch exogen zugegebenes HOCI als Testsystem eingesetzt wird. HOCI induziert in konzentrationsabhängiger Weise Apoptose, die auf der Wechselwirkung zwischen HOCI und Superoxidanionen, die zur Bildung von aggressiven Hydroxylradikalen führt, beruht (Bauer G. HOCI-dependent singlet oxygen and hydroxyl radical generation modulate and induce apoptosis of malignant cells. Anticancer Res 33: 3589-3602, 2013). Diese Apoptoseinduktion wird durch in geeigneter Konzentration exogen zugegebene SOD vollständig gehemmt. Die neutralisierende Wirkung von Single-Domain-VHH- oder Fab-Fragmenten kann also dadurch bestimmt werden, dass geprüft wird, ob die Hemmung der Apoptose durch die zu testenden Single-Domain-VHH- oder Fab-Fragmente wieder aufgehoben wird. Der quantitative Einsatz dieses Testsystems erfordert die Verknüpfung mehrerer einfacher Schritte, die dem Fachmann geläufig sind. Im ersten Schritt wird die Konzentration an kommerziell erhältlicher HOCI (oder Natriumhypochlorid, das in Medium ausreichend HOCI bildet) bestimmt, die innerhalb einer angemessenen Versuchszeit (1-2 Stunden) ein signifikantes, quantifizierbares Apoptosesignal generiert. Erfahrungsgemäß sind HOCI-Konzentrationen zwischen 0.1 und 1 mM gut geeignet. Als Messgröße eignet sich der Prozentsatz apoptotischer Zellen (direkt bestimmt durch Phasenkontrastmikroskopie) oder der (3-(4,5-Dimethylthiazol-2-yl)-2,5-diphenyltetrazoliumbromid (MTT)-Test, der kommerziell erhältlich ist und der die Zellviabilität über die Messung der mitochondrialen Stoffwechselaktivität bestimmt. In einem zweiten Schritt wird die Konzentration an SOD bestimmt, die gerade ausreichend ist, die Wirkung der gewählten Konzentration an HOCI vollständig zu hemmen. Erfahrungsgemäß ist dazu eine SOD-Konzentration zwischen 5 und 50 U/ml geeignet. Der Genauigkeit dieser Bestimmung kommt eine besondere Bedeutung zu, weil sich die Hemmung der HOCI-Wirkung durch Cu/Zn-SOD als Optimimumskurve darstellt und das Erreichen der optimalen Hemmwirkung für die Aussagekraft des Tests essentiell ist. Sobald die ersten beiden Schritte zufriedenstellend ausgeführt worden sind, kann der eigentliche Test der Hemmwirkung der Single-Domain-VHH- oder Fab-Fragmente erfolgen. Dazu werden ausgewählte Konzentrationen der zu testenden Fragmente mit der vorher in Schritt #2 bestimmten optimalen Konzentration SOD für 20 Minuten vorinkubiert. Anschließend wird dieses Gemisch zu den Testzellen gegeben. Danach erfolgt die Zugabe von HOCI und die Bestimmung der Apoptoseinduktion. Optimale Hemmung der SOD und damit ein eindeutiges Ergebnis für neutralisierende Wirkung liegt dann vor, wenn die Hemmwirkung der SOD zu mindestens 90 % aufgehoben wurde, also eine Apoptoseinduktion erzielt wird, die nur um 10 % niedriger ist als die Apoptoseinduktion ohne zugegebene SOD. Wird eine Hemmwirkung auf die SOD erzielt, die dadurch charakterisiert ist, dass wenigstens 50 % der Apoptoseinduktion des Vergleichswertes ohne SOD erzielt werden, liegt eine signifikante Hemmung der SOD vor. Die Bedeutung eines solchen Befundes kann durch Erhöhung der Single-Domain-VHH- oder Fab-Fragment-Konzentration in einem Folgeversuch weiter überprüft werden, da im Falle einer signifikanten Hemmung der SOD bei Konzentrationserhöhung des Single-Domain-VHH- oder Fab-Fragmentes eine Steigerung der Wiederherstellung der Apoptoseinduktion zu erwarten ist.

Für die Bestimmung der neutralisierenden Wirkung von Single-Domain-VHH- oder Fab-Fragmenten, die gegen Katalase gerichtet sind, eignet sich ein Testsystem, das auf der Apoptose-auslösenden Wirkung von H₂O₂ und dem Abbau von H₂O₂ durch Katalase beruht. Als zelluläres Testsystem können dabei sowohl nichtmaligne als auch maligne Zellen eingesetzt werden. Voraussetzung ist lediglich, dass in dem gewählten Zellsystem Apoptoseinduktion quantitativ bestimmbar ist. Als H₂O₂-Quelle ist H₂O₂-generierende Glucoseoxidase (GOX) der direkten Zugabe von H₂O₂ vorzuziehen, weil die stetige Produktion von H₂O₂ durch GOX (unter Verwendung von Glucose aus dem Nährmedium der Zellen) eine sehr präzise Einstellung des H₂O₂-Fluxes erlaubt. Das Testsystem beruht auf der durch Katalase vermittelten Hemmung der Apoptoseinduktion durch GOX und der Wiederherstellung der Apoptoseinduktion durch Katalase-neutralisierende Single-Domain-VHH- oder Fab-Fragmente. In einem ersten Schritt wird die Konzentration an GOX bestimmt, die innerhalb einer angemessenen Zeit (1-2 Stunden) signifikant Apoptose induziert. Dazu sind in der Regel, je nach Zellsystem, GOX-Konzentrationen zwischen 0.1 und 40 mU/ml notwendig. In einem zweiten Schritt wird die Konzentration an humaner Katalase bestimmt, die für die Aufhebung des durch GOX erzielten Effektes gerade ausreichend ist. Der dazu notwendige Konzentrationsbereich liegt erfahrungsgemäß im Bereich von weniger als 20 U/ml Katalase. Nach erfolgreicher Bestimmung der geeigneten Konzentrationen an GOX und Katalase kann dann das eigentliche Experiment zur Bestimmung der Katalase-neutralisierenden Wirkung von Single-Domain-VHH- oder Fab-Fragmenten durchgeführt werden. Dazu wird die gewählte Katalasekonzentration mit ausgewählten Konzentrationen der zu testenden Single-Domain-VHH- oder Fab-Fragmente für 20 Minuten vorinkubiert. Danach wird dieses Gemisch zusammen mit der als geeignet bestimmten Konzentration GOX zu den Zellen gegeben und die Apoptoseinduktion gemessen. Eine sichere neutralisierende Wirkung der Single-Domain-VHH- oder Fab-Fragmente liegt dann vor, wenn die Hemmwirkung der Katalase auf die GOX-vermittelte Apoptoseinduktion mindestens um 90 % aufgehoben wurde, wenn also 90 % des Apoptoseinduktionswertes erreicht werden, der sich ohne Katalasezugabe in Kontrollansätzen zeigt. Wird eine Aufhebung der Hemmwirkung der Katalase von wenigstens 50 % erreicht, darf von einer signifikanten Hemmwirkung des getesteten Single-Domain-VHH- oder Fab-Fragmentes ausgegangen werden. Durch Erhöhung der Konzentration dieses Fragmentes in Folgeversuchen kann eine weitere Überprüfung dieses Befundes erzielt werden.

Als Kontrolle in den hier aufgeführten Testverfahren werden Antigen bindende Konstrukte, die an ein völlig anderes Antigen binden, eingesetzt. Dann werden im eigentlichen Test, wie weiter oben im Detail ausgeführt, die jeweils zu untersuchenden Konstrukte zu der Reaktionsmischung zugegeben und es wird gemessen, ob die enzymatische Aktivität der Katalase oder der Superoxiddismutase inhibiert oder nicht inhibiert wird. Für diese Feststellung können verschiedene Testmodelle verwendet werden, die einem Durchschnittsfachmann ohne weiteres geläufig sind. Wichtig ist diese Feststellung, um bestimmen zu können, in welcher Art und Weise das Antigen bindende Konstrukt an das Zielenzym (Katalase bzw. SOD) bindet.

Es wird davon ausgegangen, dass in den meisten Fällen, in denen das Antigen bindende Konstrukt nicht am katalytischen Zentrum oder in dessen Nähe bindet, die enzymatische Aktivität nicht oder nicht wesentlich inhibiert wird. Wenn aber das Antigen bindende Konstrukt direkt am katalytischen Zentrum oder in dessen sterischer Nähe bindet, oder aber trotz Bindung entfernt vom katalytischen Zentrum die Konformation des Zielenzyms über allosterische Wirkung so verändern, dass die normale Reaktion des Enzyms nicht mehr ungehindert ablaufen kann, können die Reaktanten nicht mehr mit dem Enzym reagieren und die Umsetzung kann nicht mehr ungehindert erfolgen. Da die Bestimmung von absoluten Werten bei biochemischen Systemen immer variiert, werden derartige Tests möglichst in einem Testansatz durchgeführt, wobei Zellen im selben Zustand und in selber Menge den unterschiedlichen Testbedingungen ausgesetzt werden, um eine Aussage treffen zu können. Im Sinn der vorliegenden Erfindung liegt eine Inhibierung der Superoxiddismutase und/oder der Katalase dann vor, wenn die katalytische Aktivität des jeweiligen Enzyms (oder die Gesamtaktivität der jeweiligen Enzympopulation) durch die Zugabe einer geeigneten Menge des Antigen bindenden Konstrukts um wenigstens 50 %, bevorzugt um wenigstens 70 % und besonders bevorzugt um wenigstens 90 % reduziert wird.

In einer weiteren Ausführungsform werden Antigen bindende Konstrukte, bevorzugt Nanobodies, zur Verfügung gestellt, die an Katalase und/oder Superoxiddismutase binden und, die diese Enzyme nicht oder nur teilweise inhibieren. Auch diese Antigen bindenden Konstrukte können vorteilhafterweise verwendet werden, indem diese Konstrukte beispielsweise so ausgestaltet sind, dass sie mit einem der Markierung dienenden Bestandteil verbindbar sind oder verbunden sind. Dadurch können beispielsweise Tumorzellen markiert werden, wenn es sich hierbei um einen Marker handelt, der in einem geeigneten Nachweissystem ein entsprechendes Signal liefert. Alternativ können diese Konstrukte auch mit einem zytotoxischen Mittel verbunden sein oder so ausgestaltet sein, dass sie mit diesem zytotoxischen Mittel verbunden werden können oder sich gegebenenfalls innerhalb des Zielorganismus mit diesem zytotoxischen Effektor verbinden.

In einer weiteren bevorzugten Ausführungsform werden Mischungen eingesetzt, wobei manche Antigen bindende Konstrukte die Katalase und/oder die Superoxiddismutase inhibieren und andere Konstrukte die Superoxiddismutase und/oder Katalase nicht oder nur teilweise inhibieren.

Die erfindungsgemäßen Antigen bindenden Single-Domain-VHH-Fragmente sind vorzugsweise gentechnologisch hergestellt und weisen nicht, wie komplette Antikörper, einen F_{c}-Teil auf. Von klassischen Fab-Fragmenten unterscheiden sie sich durch das Fehlen von leichten Ketten. Es handelt sich also nur um die Antigen bindenden Teile der schweren Kette eines Antikörpers.

Im Rahmen der vorliegenden Erfindung wurden verschiedene bevorzugte Antigen bindende Fragmente gentechnologisch hergestellt und sequenziert. Die Sequenzen sind in der vorliegenden Anmeldung offenbart. Besonders wichtig für die Antigen bindenden Sequenzen sind die CDR-Bereiche der Konstrukte. Die sogenannten "complementary determining regions" (im Folgenden kurz CDR) sind ganz bestimmte Teile der variablen Ketten der Immunglobuline. Diese CDR-Bereich sind innerhalb der Gerüstsequenz von Immunglobulinen eingebettet, bestimmen deren Spezifität und stellen den Kontakt mit dem spezifischen Antigen, an das die Immunglobuline binden, her. Die CDR-Bereiche sind die variabelsten Teile der Immunglobuline und tragen wesentlich zu der Verschiedenheit dieser Moleküle bei. Bei Immunglobulinen, die über eine schwere und eine leichte Kette verfügen, gibt es sechs CDR-Regionen. Wenn aber das Immunglobulin nur aus einer Kette besteht, wie bei den erfindungsgemäß bevorzugten Single-Domain-VHH-Fragmenten, gibt es drei CDR-Regionen.

In der Regel ist es wesentlich, dass die CDR-Regionen nahezu unverändert vorhanden sind, wenn die Bindungsspezifität beibehalten werden soll. Allerdings ist es möglich, dass geringfügige Mutationen die Funktionalität der Antikörper bindenden Konstrukte nicht negativ beeinflussen. Dies gilt insbesondere dann, wenn durch den Austausch einer Aminosäure die Struktur der CDR-Region nicht negativ beeinflusst wird. Solche Aminosäureaustausche sind dann möglich, wenn die neu eingefügte Aminosäure sehr ähnlich ist zu der ersetzten Aminosäure. In bevorzugter Ausführungsform weisen daher die Antigen bindenden Fragmente die CDR Regionen auf, die im Rahmen der vorliegenden Anmeldung offenbart wurden, oder sie unterscheiden sich höchstens in einer geringen Zahl von Aminosäuren von den jeweils offenbarten CDR Sequenzen, die die Bindungsfähigkeit und Bindungsspezifität nicht wesentlich verändert oder verringert.

Im Rahmen der vorliegenden Erfindung werden CDR-Regionen von Antigen bindenden Single-Domain-VHH-Fragmente offenbart, die von solchen Konstrukten herstammen, die entweder die Katalase und/oder Superoxiddismutase inhibieren sowie daran binden. Die erfindungsgemäßen Single-Domain-VHH-Fragmente (Nanobodies) beinhalten drei CDR-Regionen, die von Konstrukten herstammen, die Katalase oder Superoxiddismutase inhibieren.

Die erfindungsgemäßen Konstrukte können bevorzugt humanisiert werden, wenn die therapeutische Anwendung beabsichtigt ist. Dabei wird die Gerüstsequenz durch eine humane Gerüstsubstanz ersetzt oder die nicht humane Sequenz wird durch Mutationen zu einer menschlichen Sequenz verändert, wobei jedoch die Bindungseigenschaften erhalten bleiben sollen.

Für die Entwicklung von therapeutisch verwendbaren biologischen Molekülen (hier Nanobodies) sind häufig Modifikationen der Aminosäuresequenz unumgänglich. Da die Moleküle nicht vom Menschen, sondern ursprünglich von Kamelartigen stammen, ist es möglich bzw. wahrscheinlich, dass Antikörper gegen körperfremde Epitope erzeugt werden. Derartige Antikörperreaktionen würden die Wirkung des therapeutisch einzusetzenden Antikörperfragments neutralisieren. Um diese Schwierigkeiten zu vermeiden, werden die therapeutisch eingesetzten Moleküle humanisiert. Die Humanisierung von Antikörpern bzw. Antikörperfragmenten ist eine Technologie, die auf dem Fachgebiet wohlbekannt ist. Üblicherweise werden humane Gerüstsequenzen (backbone) gesucht, die mit dem ursprünglichen Molekül eine höchstmögliche Ähnlichkeit aufweisen. Dann werden die CDR-Bereiche aus dem ursprünglichen Nanobody herausgeschnitten und in die humane Sequenz transplantiert. Dabei bleibt es nicht aus, dass gewisse Anpassungen von Aminosäuresequenzen erfolgen müssen.

Ein wesentlicher Aspekt ist, dass die Stelle auf dem Antigen, an das der bindende Teil des Antikörpers bindet, durch die CDR-Sequenzen definiert ist. Im Rahmen der Humanisierung kann es erforderlich werden, bei einem oder zwei der drei CDR-Sequenzen geringfügige Modifikationen durchzuführen, damit die vorteilhaften Eigenschaften des Antigen-bindenden Teils erhalten bleiben. Daher sind die erfindungsgemäßen Single-Domain-VHH-Fragmente dadurch gekennzeichnet, dass sie wenigstens eine der CDR-Sequenzen, bevorzugt zwei und ganz besonders bevorzugt drei CDR-Sequenzen, wie in der vorliegenden Anmeldung offenbart, beinhalten.

Gegenstand der vorliegenden Erfindung sind daher Single-Domain-VHH-Fragmente, die drei der nachfolgend durch die SEQ ID-Nummern gekennzeichneten CDR-Regionen aufweisen. Es handelt sich hierbei um die Sequenzen mit den SEQ ID-Nummem 19, 20, 21 und 25, 26, 27. Weitere CDR-Sequenzen, abgeleitet aus Klonen, die an SOD binden, sind die CDRs mit den SEQ ID-Nummern 34, 35, 36.

In einer weiteren bevorzugten Ausführungsform können die erfindungsgemäßen Konstrukte weiterhin mit einem Marker versehen werden. Für diesen Einsatz ist es nicht notwendig, dass die Konstrukte das Zielenzym auch in seiner Funktion inhibieren. Es genügt vielmehr eine spezifische Bindung mit ausreichender Affinität. Eine derartige Bindung kann in einem klassischen ELISA-System gemessen werden, bei dem entweder humane Katalase oder SOD an einen geeigneten Träger gebunden ist und der Komplex aus Enzym und Single-Domain-VHH-Fragment durch ein gängiges Nachweisverfahren detektiert wird. Ein Marker kann einerseits dazu Verwendung finden, die gewünschten Tumorzellen zu markieren, um hier zu diagnostischen Aussagen zu gelangen. Andererseits kann der Marker aber auch dazu dienen, die Tumorzellen für andere Effektoren zu kennzeichnen. Es können beispielsweise als Marker solche Konstrukte eingesetzt werden, die sich mit Effektoren verbinden, die die Apoptose der Tumorzellen bewirken. Ein Beispiel für einen derartigen Marker ist ein Anti-CD3-Teil, der mit dem CD3-Rezeptor von zytotoxischen T-Zellen reagiert. Ein solcher Anti-CD3-Teil kann beispielsweise ein Antigen bindender Teil eines gegen den CD3-Rezeptor gerichteten Antikörpers sein.

In weiteren Ausführungsformen der vorliegenden Erfindung kann ein Antigen bindendes Konstrukt der vorliegenden Erfindung auch so ausgestaltet sein, dass es mit einem zytotoxischen Mittel verbunden werden kann. Als zytotoxische Mittel sind beispielsweise verschiedene Toxine bekannt, die aus verschiedenen Quellen, beispielsweise Bakterien, Pilzen oder Pflanzen herstammen. Es kann sich hierbei bevorzugt um das Cholera-Toxin, das Botulinum-Toxin oder Streptolysin handeln, um nur einige zu nennen.

In einer weiteren Ausführungsform können die erfindungsgemäßen Antigen bindenden Konstrukte mit einem zytotoxischen Mittel über verschiedene Bindungsarten und -möglichkeiten verbunden werden. Hierbei kann es sich um feste chemische Bindungen, wie kovalente Bindungen ebenso handeln, wie um ionische Wechselwirkungen oder um van der Waals-Kräfte.

In einer weiteren Ausführungsform ist es auch möglich, dass ein erfindungsgemäßes Antigen bindendes Konstrukt mit einem radioaktiven Isotop verbindbar ist oder mit diesem fest verbunden ist. Das radioaktive Isotop kann entweder dem diagnostischen Nachweis der Tumorzellen dienen oder die zytotoxische Wirkung des erfindungsgemäßen Antigen bindenden Konstrukts dadurch verstärken, dass ein radioaktives Isotop in unmittelbare Nähe zu einer Tumorzelle gebracht wird. Bevorzugt werden bei der therapeutischen Anwendung solche Isotope eingesetzt, die nur relativ kurz strahlen, um die Nebenwirkungen möglichst gering zu halten. Außerdem sollte die Halbwertszeit des Isotops relativ kurz sein, um die Belastungen des Körpers und der Umwelt im annehmbaren Bereich zu halten. Bevorzugt eingesetzt werden Yttrium-90, Rhenium-186 oder Erbium-169.

In einer weiteren Ausführungsform können die erfindungsgemäßen Antigen bindenden Konstrukte mit einem Farbstoff über verschiedene Bindungsarten und -möglichkeiten verbunden werden. Hierbei kann es sich um feste chemische Bindungen, wie kovalente Bindungen ebenso handeln, wie um ionische Wechselwirkungen oder um van der Waals-Kräfte. Der Farbstoff kann durch unterschiedliche gängige Verfahren nachweisbar sein. Derartige Konstrukte sollten für diagnostische Zwecke einsetzbar sein.

In einer weiteren Ausführungsform werden pharmazeutische Zusammensetzungen offenbart, die wenigstens ein erfindungsgemäßes Antigen bindendes Konstrukt beinhalten. Diese pharmazeutischen Zubereitungen werden bevorzugt zur Therapie von Tumorerkrankungen, ganz besonders zur Therapie von Magenkarzinom, verwendet.

Die erfindungsgemäßen pharmazeutischen Zusammensetzungen beinhalten wenigstens ein Antigen bindendes Konstrukt, das spezifisch an die Katalase bindet und diese inhibiert. In einer anderen Ausführungsform beinhalten die erfindungsgemäßen pharmazeutischen Zusammensetzungen wenigstens ein Antigen bindendes Konstrukt, das spezifisch an die Superoxiddismutase bindet und diese inhibiert.

Es ist überraschend und war nicht vorhersagbar, dass der Synergieeffekt der Hybridmoleküle ausschließlich den NO/Peroxynitritweg reaktiviert, während anti-SOD alleine ebenfalls diesen Weg reaktiviert, anti-Katalase jedoch den HOCI-Weg induziert. Die ausschließliche Reaktivierung des NO/Peroxynitritwegs durch die Hybridmoleküle stellt einen Vorteil für zukünftige Anwendungen dar, weil sich der weniger komplexe NO/Peroxynitritweg durch zusätzliche Modulation des NO-Stoffwechsels weiter optimieren lassen sollte, insbesondere im Hinblick auf eine wünschenswerte Breite der Plateauphase der Dosiswirkungsbeziehung.

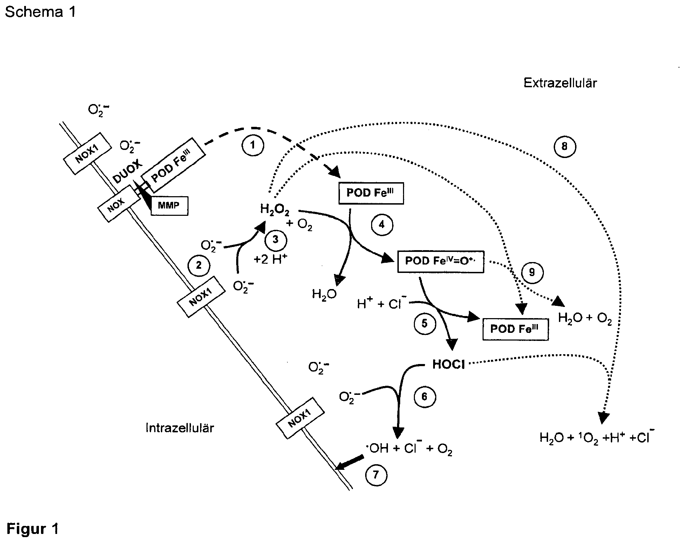

Ein weiterer Vorteil der ausschließlichen Reaktivierung des NO/Peroxynitritweges durch Anti-SOD bzw. Hybridmoleküle aus Anti-SOD und Anti-CAT liegt darin, dass aufgrund der Signalchemie (Abb. 1-4) bei Ablauf des NO/Peroxynitritwegs kein freies H₂O₂ vorliegen sollte. Dies ist deshalb wünschenswert, weil H₂O₂ eine proliferationsstimulierende Wirkung auf überlebende Tumorzellen hat, der der therapeutischen Wirkung abträglich ist.

In einer weiteren bevorzugten Ausführungsform der vorliegenden Erfindung wird wenigstens ein Antigen bindendes Konstrukt der vorliegenden Erfindung zusammen mit einem Wirkstoff eingesetzt, der Antitumoraktivität aufweist. Verschiedene Wirkstoffe, die Antitumoreffekte aufweisen, sind bekannt. Insbesondere können als Chemotherapeutika, die bei der vorliegenden Zusammensetzung auch vorhanden sein können, Stoffe genannt werden wie Taxol, Cisplatin, Endostatin, Oxaliplatin, Etopside oder Colchicin, um nur einige wenige Wirkstoffe beispielhaft zu nennen. Bevorzugt wird Taxol eingesetzt.

Bei den erfindungsgemäßen Antigen bindenden Konstrukten handelt es sich um Single-Domain-VHH-Fragmente, die gentechnologisch hergestellt werden können.

Eine genaue molekulare Anordnung der einzelnen Fragmente in dem Antigen bindenden Konstrukt ist von untergeordneter Bedeutung, solange die gewünschte Funktion, nämlich die Bindung und Inhibierung an Katalase und/oder Superoxiddismutase gewahrt bleibt.

Die erfindungsgemäßen Antigen-bindenden Konstrukte werden mit molekularbiologischen Mitteln hergestellt. Es handelt sich nicht um natürlich vorkommende Antikörper oder hieraus durch einfache (enzymatische) Spaltung hergestellte Fab-Fragmente. Zum besseren Verständnis darf kurz der Aufbau von Antikörpern rekapituliert werden. Antikörper, beispielsweise vom IgG-Typ, bestehen aus zwei Fab-Fragmenten und einem Fc-Fragment. Jedes Fab-Fragment besteht aus einer leichten und einer schweren Kette, wobei die schwere Kette in einen variablen Teil (V_{H}) und einen konstanten Teil (C_{H1}) und die leichte Kette in einen variablen Teil (V_{L}) und einen konstanten Teil (C_{L}) unterteilt werden kann. Besonders interessant sind die variablen Teile V_{H} und V_{L}, die wiederum die für die Antigen-Bindung relevanten CDRs (Complementarity Determining Regions) 1-6 beinhalten. Die Bindungseigenschaften der Fab-Fragmente werden durch die CDR-Regionen bestimmt, die in eine Gerüststruktur eingebettet sind, die die einzelnen CDR-Regionen in die räumliche Anordnung bringen.

Bei den Antigen binden Konstrukten handelt es sich um Single-Domain-VHH-Fragmente (Nanobodies). Nanobodies beinhalten nur die für die Bindung relevanten Teile der schweren Kette der Antikörper und sind sehr gut in Bakterienzellen exprimierbar (Muyldermans S. Nanobodies: Natural single-domain antibodies. Ann. Rev. Biochem. 82: 775-797, 2013).

Mit Hilfe der verschiedenen Methoden der Gentechnologie können eine Vielzahl von Antigen bindenden Konstrukte hergestellt werden. Die dabei verwendeten Methoden sind äußerst vielfältig und dem Durchschnittsfachmann wohlbekannt. Üblicherweise wird dabei so vorgegangen, dass Labortiere (Mäuse, Ratten, Kaninchen, Hühner oder Kamele, Alpakas usw.) mit dem gewünschten Antigen immunisiert werden. Da Kamele und Alpakas neben konventionellen Antikörpern von Natur aus IgG besitzen, das ausschließlich aus schweren Ketten aufgebaut ist, führt die Verwendung dieser Tiere in Kombination mit etablierten selektiven Screening-Methoden zur Gewinnung von Single-Domain-Antikörperkodierenden Nukleinsäuresequenzen. Aus geeigneten Immunzellen (beispielsweise B-Zellen) können dann Nukleinsäuresequenzen isoliert werden, die mit geeigneten Methoden, beispielsweise mit dem sogenannten Phage Display, weiter optimiert werden. Mit diesen Verfahren werden dann Antigen bindende Konstruktmoleküle gewonnen, die spezifisch an das gewünschte Antigen binden. Spezifisch heißt in diesem Zusammenhang, dass die Konstrukte möglichst nur an das gesuchte Molekül, genauer gesagt nur an ein Epitop dieses Moleküls (SOD bzw. Katalase) binden. Unspezifische Kreuzreaktionen sind in der Regel unerwünscht.

Eine andere wichtige Eigenschaft dieser Antigen bindenden Konstrukte ist, dass diese sensitiv an das gewünschte Antigen binden. Sensitiv heißt, dass schon bei einer sehr geringen Konzentration des Antigen bindenden Konstrukts eine spezifische Bindung an das gewünschte Antigen bzw. das gewünschte Epitop erfolgt. Vereinfacht ist ein Antigen bindendes Konstrukt umso sensitiver je besser es an das Zielantigen bindet. Da die Single-domain-VHH-Fragmente nicht wie klassische Fab-Fragmente ein bestimmtes Epitop umschließen, sondern aufgrund ihrer molekularen Struktur an räumliche Vertiefungen des Antigens binden, ergeben sich wesentliche Unterschiede bezüglich der Erfassbarkeit bestimmter Epitope durch diese beiden Arten von Single-Domain-VHH-Fragmenten.

Die erfindungsgemäßen Antigen-bindenden Konstrukte binden spezifisch an die Superoxiddismutase und inhibieren dieses Enzym. Die Inhibierung des Zielmoleküls Superoxiddismutase erfolgt dadurch, dass das Antigen bindende Konstrukt entweder am katalytisch aktiven Zentrum der Superoxiddismutase (SOD) bindet oder in der Nähe dieses katalytischen Zentrums, wodurch eine sterische Hemmung des Enzyms erfolgt. Superoxidanionen (das übliche und spezifische Substrat der SOD) können dann nicht mehr an das Enzym binden und von ihm katalytisch zu H₂O₂ umgesetzt werden..

Entsprechendes gilt für die Katalase. Die erfindungsgemäßen Antigen bindenden Konstrukte binden spezifisch und sensitiv an die Katalase und inhibieren diese, so dass die enzymatische Umwandlung von H₂O₂ zu H₂O + 1/2 O₂, bzw. von Peroxynitrit zu NO₂⁻ und 1/2 O₂ gehemmt ist und die Oxidation von NO durch das aktive Intermediat "Compound I" der Katalase verhindert wird In der vorliegenden Anmeldung wurden die in der nachfolgenden Liste aufgeführten Abkürzungen verwendet:
- AEBSF: 4-(2-Aminoethyl)-benzenesulfonyl fluoride (Hemmstoff der NADPH-Oxidase)
- 3-AT: 3-Aminotriazol (Katalasehemmstoff)
- Anti-CAT: Antikörper gegen Katalase
- Anti-SOD: Antikörper gegen SOD
(Aus Platzgründen werden in den Abbildungen die Bezeichnungen Anti-CAT und Anti-SOD durch aCAT und aSOD ersetzt)
- CAT: Katalase
- Compound I: Aktivierte Zwischenstufe, sowohl von Katalase mit der Formel CAT Fe^{IV} = O⁺· als auch Peroxidase, mit der Formel POD Fe^{IV} = O·⁺. Compound I wird bei der Reaktion von Katalase oder Peroxidase mit einem Molekül Wasserstoffperoxid gebildet. Katalase kann Compound I auch mit einem Molekül Peroxynitrit bilden.
- Duox: Duale Oxidase (membranständiges Enzym das aus einer NADPH-Oxidase- und einer Peroxidasedomäne besteht. Die Peroxidasedomäne wird mithilfe von Proteasen abgespalten)
- FBS: Fetales Bovines Serum (Foetales Kälberserum)
- FeTPPS: 5-, 10-, 15-, 20-Tetrakis(4-sulfonatophenyl)porphyrinato iron(III) chloride (Peroxynitrite decomposition catalyst)
- NO: Nitric oxide (Stickstoffmonoxid)
- NOD: Nitric oxide dioxygenase (oxidiert NO zu Nitrat)
- NOS: NO-Synthase
- NOX: NADPH-oxidase (hier insbesondere die membranständige NOX-1)
- POD: Peroxidase (in diesem Kontext kommt insbesondere die Fähigkeit bestimmter Peroxidasen zum Tragen, die in Gegenwart von Wasserstoffperoxid Chlorid zu HOCI oxidieren können)
- PON: Peroxynitrit
- POR: Cytochrom P 450 Oxidoreduktase
- RAS, RAC: Onkogene
- ROS: Reactive oxygen and nitrogen species (Radikalische und nichtradikalische Spezies wie Superoxidanionen, Hydroxylradikale, Stickstoffmonoxid, Wasserstoffperoxid, HOCI, Peroxynitrit, etc.)
- siRNA: small interfering RNA (Reagenz zum spezifischen Herunterregulieren der Synthese definierter Genprodukte)
- SOD: Superoxiddismutase (hier insbesondere SOD-1 (Cu⁺⁺ im aktiven Zentrum der Tumorzellen und MnSOD aus Bakterien für analytische Zwecke)
- TGF-beta: Transforming growth factor type beta

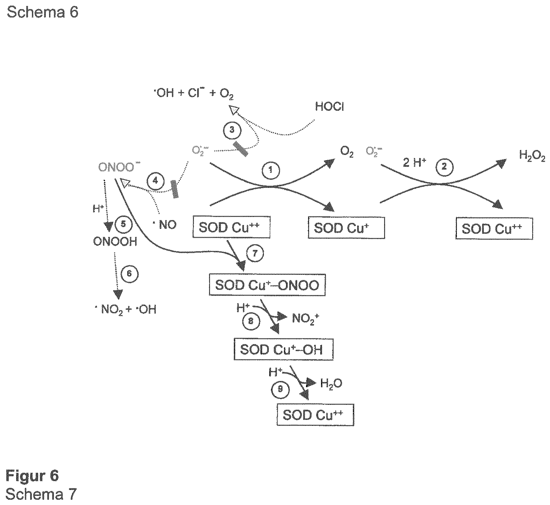

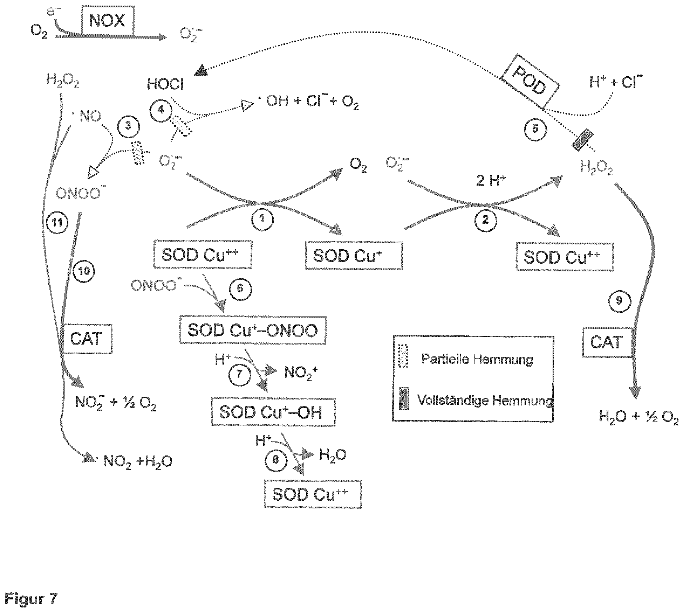

Die nachfolgenden Schemazeichnungen fassen zunächst die Apoptose-induzierenden ROS-Signalwege in transformierten Zellen zusammen (Schema 1: HOCI-Weg; Schema 2: NO/Peroxynitritweg) und zeigen dann die Tumorzell-spezifische Wirkung membranständiger Katalase und SOD auf diese Signalwege (Schema 3 und 4).
Figur 1: Schema 1 (HOCI-Signalweg) zeigt die Membran einer maligne transformierten Zelle, die durch Expression von NOX1 und DUOX (bestehend aus einer mit NOX1-verwandten NOX-Domäne und einer Peroxidasedomäne) gekennzeichnet ist. Dabei stellt die Expression von NOX1 ein spezifisches Merkmal maligner Zellen dar, während sich DUOX auch in Normalzellen nachweisen lässt. Durch die Wirkung einer Matrix-Metalloprotease (MMP) wird die Peroxidasedomäne freigesetzt ①. Die durch NOX1 generierten Superoxidanionen ② dismutieren zu H₂O₂ ③, welches von der Peroxidase als Substrat genutzt wird ④. Dabei entsteht aus der nativen Peroxidase (PODFe^{III}) das reaktive Intermediat "Compound I" (PODFe^{IV}=O⁺), welches in der Lage ist, Chloridionen zu HOCI zu oxidieren ⑤. Das im mikromolaren Konzentrationsbereich vorliegende HOCI entwickelt nur dann eine toxische Wirkung, wenn es mit Superoxidanionen unter Bildung Apoptose-auslösenden Hydroxylradikale reagiert ⑥. Der entscheidende Schritt ist dabei die Lipidperoxidation durch Hydroxylradikale ⑦, die über die Bildung von Ceramiden zur Auslösung des mitochondrialen Wegs der Apoptose führt (nicht gezeigt). Liegt ein relativer Überschuss von H₂O₂ gegenüber Peroxidase vor, können die unter ⑧ und ⑨ gezeigten Reaktionen durch Verbrauch von HOCI ⑧ oder Verhinderung seiner Synthese ⑨ zu einem Abbruch des HOCI-Signalwegs führen.

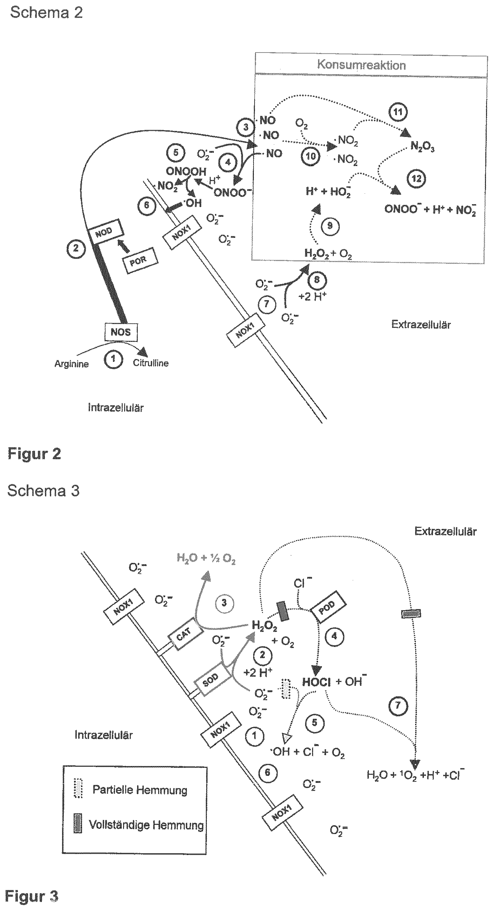

Figur 2: Schema 2 zeigt den NO/Peroxynitrit-Signalweg transformierter Zellen. NO Synthase (NOS) generiert Stickstoffmonoxid (NO) ①. Ein nicht unbeträchtlicher Teil des NO wird durch NO-Dioxygenase (NOD) zu Nitrat umgesetzt ②. Dabei wird NOD durch Cytochrom P450-abhängige Oxidoreduktase (POR) gesteuert. NO zeigt hohe Membrangängigkeit ③ und kann mit an der Außenseite der transformierten Zelle generierten Superoxidanionen zu Peroxynitrit (ONOO⁻) reagieren ④. Die durch Protonierung von Peroxynitrit entstehende Peroxynitritsäure (ONOOH) ⑤ zerfällt extrem schnell in NO₂ und Apoptose-auslösende Hydroxylradikale ⑥. Die Reaktionsfolge ⑦ - ⑫ zeigt eine alternative Reaktionsmöglichkeit für NO, die insgesamt eine Konsumreaktion darstellt und den NO/Peroxynitritweg schwächen oder aufheben kann. Diese Konsumreaktion stellt jedoch auch eine Möglichkeit dar, durch Erhöhung der NO-Konzentration H₂O₂-abhängige Prozesse zu modulieren.
Figur 3: Schema 3 zeigt, dass Tumorzellen durch Expression membranständiger Katalase (CAT) den HOCI-Signalweg ①, ②, ④ - ⑥ wirkungsvoll unterbinden, indem durch Zerstörung von H₂O₂ ③ die HOCI-Synthese verhindert wird. Membranständige SOD fördert zwar die Dismutation von Superoxidanionen zu H₂O₂, was den HOCI-Weg beeinflussen könnte, doch kommt dieser Effekt durch die Wirkung der Katalase, die dieses H₂O₂ abbaut, nicht zum Tragen. Das SOD-vermittelte Absenken der Superoxidanionenkonzentration hemmt jedoch die für den Signalweg essentielle Interaktion zwischen HOCI und Superoxidanionen ⑤ und steigert dadurch den Schutz der Tumorzelle vor ROS-Signaling. Die Analyse der Hemmwirkungen zeigt, dass die auf der Membran von Tumorzellen befindliche Katalase für eine vollständige und dominante Hemmung der HOCI-Synthese ausreicht, während die SOD-Wirkung alleine zwar einen deutlich messbaren, jedoch nur partiellen Schutz bietet.

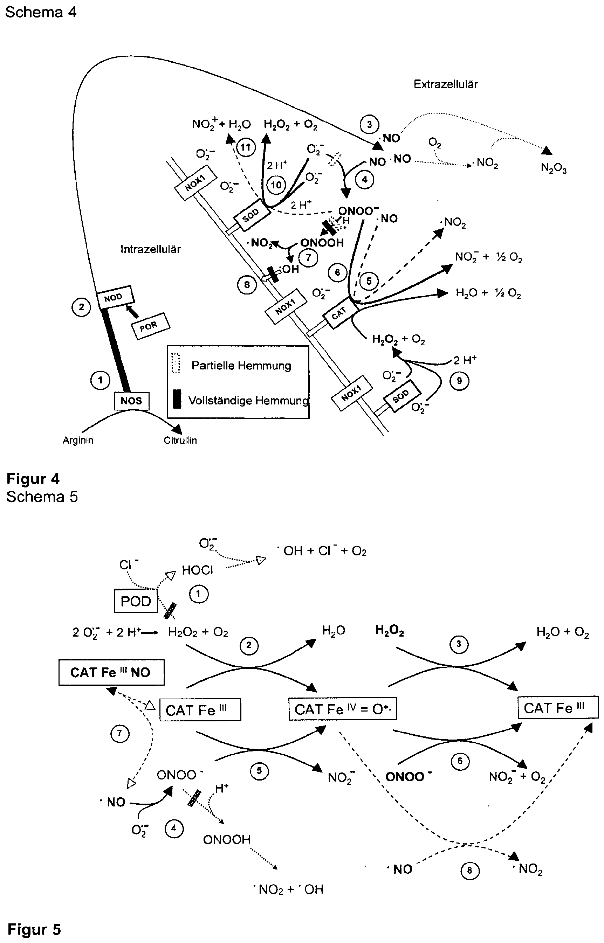

Figur 4: Schema 4 fasst das komplexe Zusammenspiel von membranständiger Katalase und SOD beim Schutz von Tumorzellen vor dem NO/Peroxynitritsignalweg zusammen. Katalase verhindert Peroxynitritbildung durch Oxidation von NO zu NO₂ ⑤ und zerstört eventuell entstehendes Peroxynitrit durch Abbau ⑥. SOD verhindert Peroxynitritbildung durch das Wegfangen von Superoxidanionen ④ und ist ebenfalls in begrenztem Maße zur Zerstörung von Peroxynitrit ⑪ fähig. Außerdem darf spekuliert werden, dass das durch SOD in Reaktion ⑩ gebildete H₂O₂ durch die in Schema 2 gezeigte Konsumreaktion zu einer zusätzlichen Senkung der NO-Konzentration beiträgt. Das Schema zeigt eindrucksvoll, dass Tumorzellen in koordinierter Weise zu einer mehrfachen Kontrolle des NO/Peroxynitritwegs befähigt sind.
   In den nachfolgenden Schemazeichnungen 5 (Fig. 5) und 6 (Fig. 6) sind die zum Verständnis der multiplen Wirkung der protektiven Enzyme Katalase und SOD notwendigen enzymatischen Details zusammengefasst.
Fig. 5: Schema 5 zeigt, dass die Hemmung des HOCI-Signalwegs ① durch Katalase über einen Zweistufenmechanismus ②, ③ erfolgt, bei dem das Katalase-Intermediat "Compound I" (CATFe^{IV}=O⁺) von zentraler Bedeutung ist und zu nativem Enzym zurückgebildet wird. Die Hemmung des NO/Peroxynitritwegs ④ erfolgt einerseits durch einen Abbau von Peroxynitrit über einen Zweistufenmechanismus unter Beteiligung von Compound I, andererseits durch Oxidation von NO ⑧. Die Oxidation von NO steht im Gleichgewicht mit einer NO-abhängigen Hemmung der Katalase, die allerdings erst bei relativ hohen NO-Konzentrationen zum Tragen kommt.
Fig. 6: Schema 6 zeigt, dass die SOD-vermittelte Dismutation von Superoxidanionen zu H₂O₂ ①, ② über einen Zweistufenmechanismus erfolgt, bei dem die Reduktion des enzymgebundenen Cu⁺⁺ und die Oxidation des Cu⁺ eine tragende Rolle spielen. Die Umsetzung von Superoxidanionen wirkt sowohl der für den HOCI-Weg wichtigen Interaktion zwischen HOCI und Superoxidanionen ③, als auch der Entstehung von Peroxynitrit ④ entgegen. SOD besitzt auch das Potenzial, Peroxynitrit über die Reaktionsschritte ⑦ - ⑨ zu zerstören.
   Schema 5 und 6 zeigen, dass Katalase und SOD entgegen der Lehrbuchmeinung nicht durch hochselektive Reaktionen charakterisiert sind, sondern vielmehr multiple, teilweise überlappende Funktionen ausführen können. Dies führt insgesamt zu einer außerordentlich plastischen und komplexen biologischen Wirkung.
Fia. 7: Schema 7 stellt den Gesamtzusammenhang zwischen den an der Außenseite von Tumorzellen agierenden Enzymen dar. Membranständige NADPH Oxidase (NOX) generiert die für die beiden Signalwege essentiellen Superoxidanionen. Membranständige SOD wandelt einen wesentlichen Teil der Superoxidanionen in H₂O₂ um ①, ②. Dies führt zu einer partiellen Hemmung der Peroxnitritbildung ③ und zur partiellen Hemmung der Interaktion zwischen Superoxidanionen und HOCI ④, wobei HOCI durch Peroxidase (POD) gebildet wurde ⑤. Zusätzlich zersetzt SOD auch Peroxynitrit (Reaktionsfolge ⑥-⑧). Katalase verhindert HOCI-Synthese durch Abbau von H₂O₂ ⑨, baut Peroxynitrit ab ⑩ und verhindert Peroxynitritbildung durch Oxidation von NO ⑪.

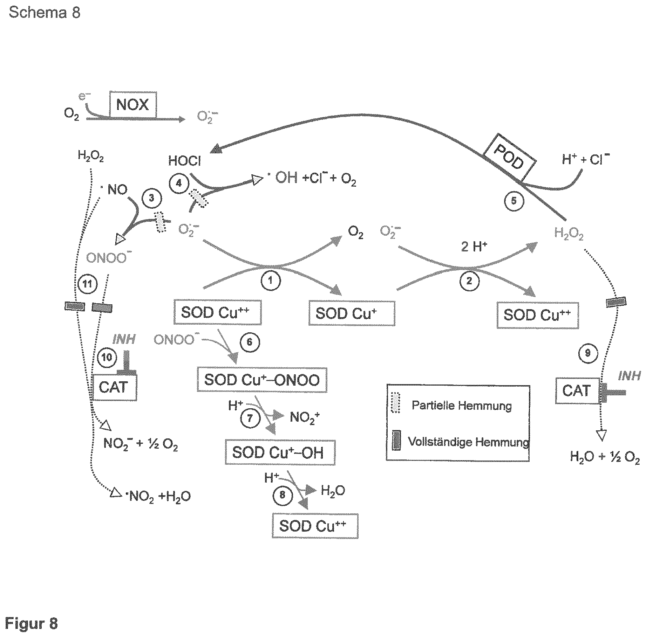

Der in der Schemazeichnung 7 zusammengefasste Sachverhalt führt zu der in Schemazeichnung 8 (Fig. 8) dargestellten Schlussfolgerung, wonach eine alleinige Hemmung der membranständigen Katalase ⑨, ⑩ zu einer Reaktivierung des HOCI-Signalwegs und des NO/Peroxynitritwegs führen kann, da SOD nur eine partielle Protektion der Schritte ③ und ④ erzielt.

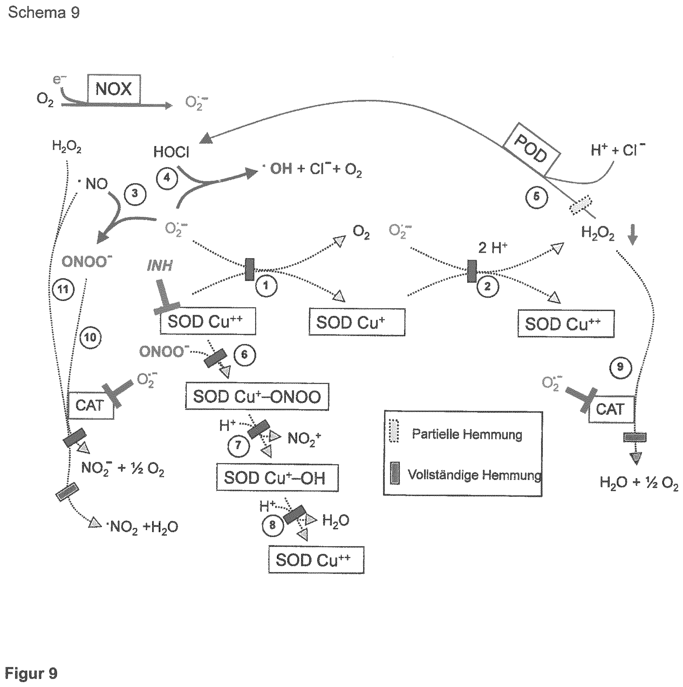

Umgekehrt würde man zunächst jedoch nicht erwarten, dass eine Hemmung der SOD zu einer Reaktivierung der Apoptose-auslösenden Signalwege ausreichen sollte, da die dominante Hemmwirkung der Katalase dem entgegenstehen sollte. Wie Schema 9 (Fig. 9) erläutert, trifft dies jedoch nicht zu. Der Schlüssel für das Verständnis dieses zunächst unerwarteten Befundes liegt in der hemmenden Wirkung von Superoxidanionen auf Katalase. Da nach Hemmung der SOD ein lokaler Anstieg der Superoxidanionenkonzentration zu verzeichnen ist (dem nur die spontane Dismutationsreaktion entgegen wirkt), wird ein indirekter Hemmeffekt auf die Katalase erreicht, wenn ausschließlich SOD direkt gehemmt wurde.

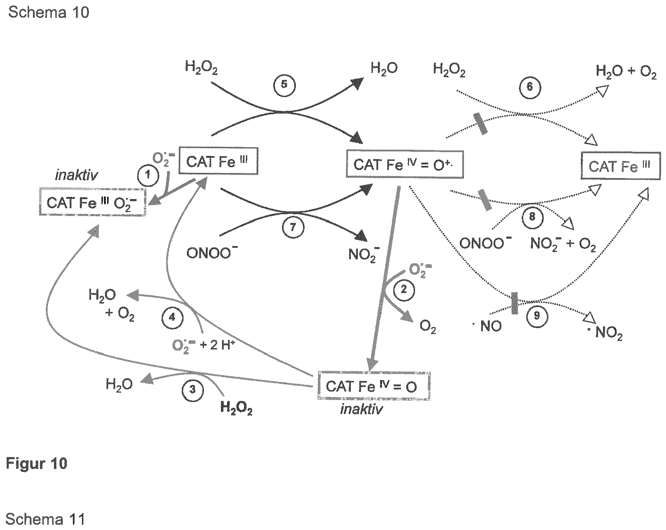

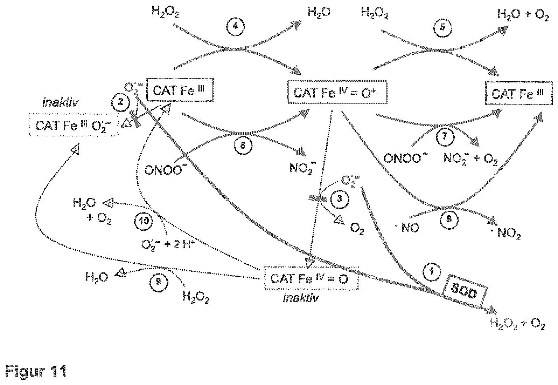

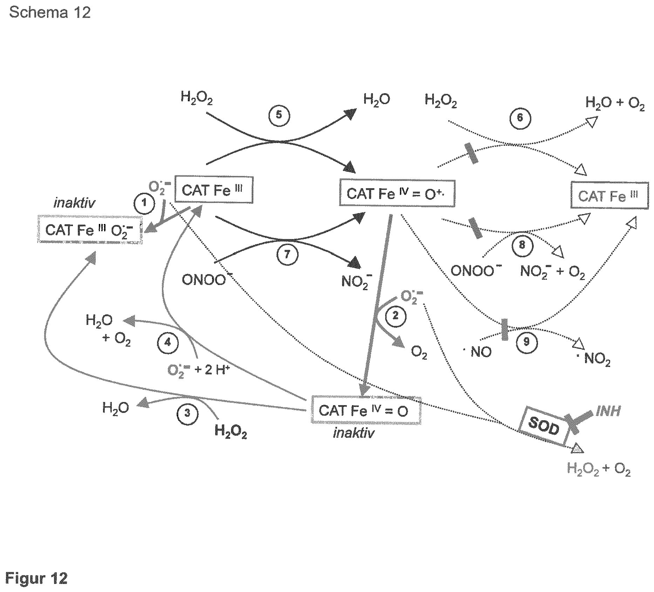

Die enzymatische Grundlage für die Superoxidanionen-vermittelte Hemmung der Katalase wird in Schema 10 (Fig. 10) erläutert. Superoxidanionen können einerseits native Katalase in das inaktive Compound III (CATFe^{III}O₂·⁻) ①, anderseits Compound I (CATFeIV=O·⁺) durch einen Ein-Elektronenübergang in das inaktive Compound II (CATFe^{IV}=O) überführen und dadurch die Enzymwirkung substantiell hemmen. Es ist daher verständlich, dass ein SOD-vermitteltes Absenken der Superoxidanionenkonzentration unter die für die Hemmungswirkung notwendige Konzentration zur Sicherung der Katalaseaktivität beiträgt (siehe Fig. 11, Schema 11). Umgekehrt wird sofort erkennbar, dass die direkte Hemmung der SOD zwangsläufig eine indirekte Hemmung der Katalase nach sich zieht (Fig. 12, Schema 12).

Die vorliegende Erfindung wird durch die nachfolgenden Beispiele näher erläutert. Die Ergebnisse der erfindungsgemäßen Experimente werden häufig in den Figuren dargestellt. Offenbart werden Sequenzen von besonders bevorzugten Ausführungsformen.

### Beispiel 1: Bereitstellung der Konstrukte und Materialien

Die nachfolgenden Beispiele wurden mit folgenden Antikörpern, Fab-Fragmenten oder Single-Domain-VHH-Fragmenten durchgeführt:
1) Monoklonaler Antikörper (Maus, IgG1) gegen humane SOD-1 (Clone SD-G6) (Chargennummer 035K4823). Hersteller Sigma Aldrich, Schnelldorf, Deutschland (als Kontrolle).
2) Rekombinantes humanes Fab-Fragment gegen humane Katalase, Format Fab-V5Sx2, hergestellt von AbDSerotec aus einer HuCAL® Library (näher beschrieben in EP 859 841 bzw. US 6,300,064). Eingesetzt wurde das Konstrukt #AbD15562 mit Katalase-Hemmwirkung (Vergleich).
3) Rekombinantes humanes Fab-Fragment gegen humane SOD, Format Fab-V5Sx2, hergestellt von AbDSerotec aus einer HuCAL® Library. Eingesetzt wurde das Konstrukt #AbD15660 mit SOD-Hemmwirkung (Vergleich).
4) Rekombinante Single Domain VHH-Fragmente gegen humane Katalase (erfindungsgemäß), hergestellt in Kooperation mit einem kommerziellen Anbieter.
   Die Herstellung erfolgte durch Immunisierung von Alpakas mit humaner Katalase (Katalase [EC 1.11.16] aus menschlichen Erythrozyten gereinigt, erhalten von Sigma (Schnelldorf), Katalognummer C 3556) unter tierärztlicher Aufsicht, Gewinnung von RNA aus den B-Zellen der Tiere, Reverser Transkription, Klonierung in E. coli und Isolierung über die Phage Display Technologie. Klone, die für Single-Domain-VHH-Fragmente kodieren, die an humane Katalase binden wurden durch das Testen von Überständen in einem geeigneten ELISA selektiert. In einer zweiten Runde wurde durch Einsatz des von Heinzelmann und Bauer beschriebenen Zellkultursystems (Heinzelmann S. and Bauer G. Multiple protective functions of catalase against intercellular apoptosis-inducing ROS signaling of human tumor cells, Biol. Chem. 391, 675-693, 2010) geprüft, welche der an Katalase bindenden Single-Domain-VHH-Fragmente tatsächlich zu einer Hemmung der Katalase führen, was sich als ROS-abhängige Apoptoseinduktion in den Zellen ausdrückt. Verwendet wurden die Single Domain VHH Fragmente aCATcb0972, aCATcb0974, die beide an humane Katalase binden und diese neutralisieren, und aCATcb0973 und aCATcb0975, die an humane Katalase binden, diese aber nicht neutralisieren. Die den Single-Domain-VHH-Fragmenten zugrundeliegenden Klone wurden durch Standardverfahren sequenziert und daraus die Aminosäuresequenz bestimmt.
   Bevorzugte Ausführungsformen der Erfindung weisen die folgenden Sequenzen auf.
   Die Sequenzen der Antigen-bindenden Fragmente wurden sowohl auf DNA wie auch auf Proteinebene analysiert und es wurden die Antigen bindenden Bereiche (CDR) bestimmt. Im Folgenden werden nur die Aminosäuresequenzen wiedergegeben. Diese Antigen bindenden Bereiche sind wesentlich für die Spezifität der Antigen bindenden Fragmente.
   In bevorzugter Ausführungsform der vorliegenden Erfindung beinhalten die Antigen bindenden Konstrukte, insbesondere die Single-Domain-VHH-Fragmente oder die Nanobodies drei CDR Sequenzen gemäß den Ansprüchen.
   Im Folgenden sind die CDR Sequenzen auf Protein-Ebene wiedergegeben. In der vollständigen Sequenz sind die jeweiligen Positionen durch Unterstreichungen angegeben.
   cb 0972 (an Katalase bindend und neutralisierend):
   **CDR Sequenz auf Protein Ebene:**
      CDR1: RTFNTYGMG (SEQ ID NO:19)
      CDR2: TISWSGDSTYYADSVKG (SEQ ID NO:20)
      CDR3: NSEYGDSY (SEQ ID NO:21)
   **cb 0973** (an Katalase bindend aber **nicht** neutralisierend; nicht erfindungsgemäß)
   **CDR Sequenz auf Protein Ebene:**
      CDR1: FIFNTYSMR (SEQ ID NO:22)
      CDR2: SISTGGYSTYADSVKG (SEQ ID NO:23)
      CDR3: GAFVRGERP (SEQ ID NO:24)
   **cb 0974** (an Katalase bindend und neutralisierend):
   **CDR Sequenz auf Protein Ebene:**
      CDR1: SIFSIASMG (SEQ ID NO:25)
      CDR2: TITSDGSTKYADSVKG (SEQ ID NO:26)
      CDR3: DADDLEPGSYDYDY (SEQ ID NO:27)
   **cb 0975** (an Katalase bindend, aber **nicht** neutralisierend; nicht erfindungsgemäß)
   **CDR Sequenz auf Protein Ebene:**
      CDR1: SIFSIYVMA (SEQ ID NO:28)
      CDR2: TVTSGGATNYANSVKG (SEQ ID NO:29)
      CDR3: EDYYDYGLSRSKIY (SEQ ID NO: 30)
5) Rekombinante Single Domain VHH-Fragmente gegen humane SOD1, hergestellt in Kooperation mit einem kommerziellen Anbieter.

Die Herstellung erfolgte durch Immunisierung von Alpakas mit humaner SOD1 (SOD1 = Cu/ZnSOD [EC 1.15.1.1] aus menschlichen Erythrozyten gereinigt, erhalten von Sigma (Schnelldorf), Katalognummer S 9636) unter tierärztlicher Aufsicht, Gewinnung von RNA aus den B-Zellen der Tiere, Reverser Transkription, Klonierung in E. coli und Isolierung über die Phage Display Technologie. Klone, die für Single-Domain-VHH-Fragmente kodieren, die an humane SOD1 binden wurden durch das Testen von Überständen in einem geeigneten ELISA selektiert. In einer zweiten Runde wurde durch Einsatz des von Heinzelmann und Bauer beschriebenen Zellkultursystems (Multiple protective functions of catalase against intercellular apoptosis-inducing ROS signaling of human tumor cells, Biol. Chem. 391, 675-693, 2010) geprüft, welche der an SOD bindenden Single-Domain-VHH-Fragmente tatsächlich zu einer Hemmung der SOD führen, was sich in dem verwendeten Tumorsystem als ROS-abhängige Apoptoseinduktion in den Zellen ausdrückt, da durch Hemmung der SOD die Konzentration freier Superoxidanionen aufgrund des Wegfalls der enzymatischen Dismutation dramatisch ansteigt und zu einer parallelen indirekten Hemmung der Katalase führt. Dies drückt sich nachfolgend als ROS-abhängige Apoptoseinduktion aus. In einem weiteren Kontrollversuch wurde die spezifische Hemmung der SOD durch rekombinante Single-Domain-VHH-Fragmente dadurch verifiziert, dass der steigernde Effekt dieser Fragmente auf Apoptoseinduktion durch exogen zugegebenes HOCI geprüft wurde, wie dies bei Bauer 2013 (HOCI-dependent singlet oxygen and hydroxyl radical generation modulate and induce apoptosis of malignant cells. Anticancer Res 33: 3589-3602, 2013) beschrieben ist. Verwendet wurden das Single-Domain-VHH Fragment aSODcb0989, das an humane SOD1 bindet und diese neutralisiert, und die Fragmente aSODcb0987 und aSODcb0991, die an humane SOD1 binden, diese aber nicht neutralisieren.

Die den Single-Domain-VHH-Fragmenten zugrundeliegenden Klone wurden durch Standardverfahren sequenziert und daraus die Aminosäuresequenz bestimmt. Bevorzugte Ausführungsformen der Erfindung weisen folgende Sequenzen auf:

### anti-SOD VHH's:

**cb 0987** (an SOD bindend, aber nicht neutralisierend; nicht erfindungsgemäß)
**CDR Sequenz auf Protein Ebene:**
   CDR1: SISEIDAMY (SEQ ID NO:31)
   CDR2: GITNDGTRYYADSVKG (SEQ ID NO:32)
   CDR3: LPNPPPGY (SEQ ID NO:33)
**cb 0989** (an SOD bindend und neutralisierend::
**CDR Sequenz auf Protein Ebene:**
   CDR1: FTISNYPMT (SEQ ID NO: 34)
   CDR2: RINSGGDRTLYADSVKG (SEQ ID NO:35)
   CDR3: SGAGWRY (SEQ ID NO:36)
**cb 0991** (an SOD bindend, aber nicht neutralisierend; nicht erfindungsgemäß)
**CDR Sequenz auf Protein Ebene:**
   CDR1: SISDIDAMY (SEQ ID NO:37)
   CDR2: GITNDGTEYFADSVKG (SEQ ID NO:38)
   CDR3: LPNPPPGY (SEQ ID NO:39)

Durch Verbindung der Klone cb 0972 (Katalase neutralisierend) und cb 0989 (SOD neutralisierend) über einen Linker wurden gentechnisch die bispezifischen Hybrid Single-Domain-VHH-Fragmente cb **1081** (anti-CATanti-SOD) und **cb 1082** (anti-SODanti-CAT) hergestellt.

### Bispezifischer anti-Katalase-SOD VHH (nicht erfindungsgemäß)

**cb 1081**
**CDR Sequenz auf Protein Ebene:**
   CAT CDR1: RTFNTYGMG (SEQ ID NO:40)
   CAT CDR2: TISWSGDSTYYADSVKG (SEQ ID NO:41)
   CAT CDR3: NSEYGDSY (SEQ ID NO:42)
   SOD CDR1: FTISNYPMT (SEQ ID NO:43)
   SOD CDR2: RINSGGDRTLYADSVKG (SEQ ID NO:44)
   SOD CDR3: SGAGWRY (SEQ ID NO:45)

### Bispezifischer anti-SOD-Katalase VHH (nicht erfindungsgemäß)

**cb 1082**
**CDR Sequenz auf Protein Ebene:**
   SOD CDR1: FTISNYPMT (SEQ ID NO:46)
   SOD CDR2: RINSGGDRTLYADSVKG (SEQ ID NO:47)
   SOD CDR3: SGAGWRY (SEQ ID NO:48)
   CAT CDR1: RTFNTYGMG (SEQ ID NO:49)
   CAT CDR2: TISWSGDSTYYADSVKG (SEQ ID NO:50)
   CAT CDR3: NSEYGDSY (SEQ ID NO:51)

Der NADPH oxidase Inhibitor 4-(2-aminoethyl-benzenesulfonyl fluoride (AEBSF), der Katalaseinhibitor 3-aminotriazole (3-AT), der HOCI-Fänger Taurin, der Singulettsauerstoff-fänger Histidin, Glucoseoxidase (GOX) wurden von Sigma (Schnelldorf, Germany) erhalten.

Peroxynitrit und der "Peroxynitrite decomposition catalyst" (funktioneller Peroxynitritfänger) 5-, 10-, 15-, 20-Tetrakis(4-sulfonatophenyl)porphyrinato iron(III) chloride (FeTPPS) wurden von Calbiochem (Merck Biosciences GmbH, Schwalbach/Ts, Germany) bezogen.

Eine genaue Beschreibung dieser Wirkstoffe findet sich in den Publikationen Heinzelmann and Bauer (2010, Multiple protective functions of catalase against intercellular apoptosis-inducing ROS signaling of human tumor cells, Biol. Chem. 391, 675-693), und Bechtel und Bauer (2009, Catalase protects tumor cells against apoptosis induction by intercellular ROS signaling, Anticancer Res 29: 4541-4557).

### Beispiel 2: Genabschaltung mit siRNAs

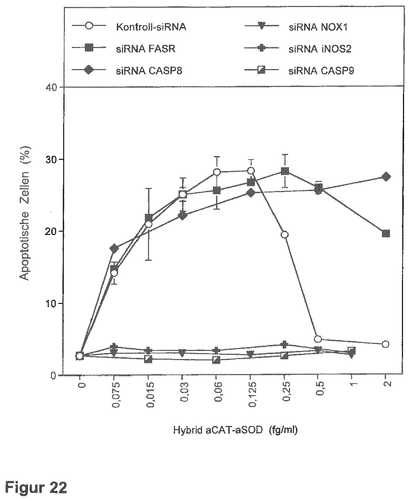

Für die in Fig. 22 beschriebene Analyse unter Einsatz des spezifischen Abschaltens von Genen mittels der siRNA-Technik wurden folgende siRNAs (erhalten von Qiagen, Hilden, Deutschland) eingesetzt:
A. Kontrol siRNA ("siCo"), (catalog no. 1022076; Sequenz:
   r(UUCUCCGAACGUGUCACGU)dTdT (sense) (SEQ ID NO:1)
   ACGUGACACGUUCGGAGAA)dTdT (antisense) (SEQ ID NO:2).
   Der Hersteller ermittelte, dass siCo die Expression keines bekannten Genes beeinflusst.
B. "High-performance validated siRNAs" für den Knockdown von:
   FAS Rezeptor ("siRNA FAS R.") (Hs_FAS_7_HP Validierte siRNA, Katalog Nr. SI02654463; Zielsequenz: AAG GAG TAC ACA GAC AAA GCC) (SEQ ID NO:3);
   Caspase-8 ("siRNA CASP8") (Hs_CASP8_11_HP Validierte siRNA; Katalog Nr SI02661946, Zielsequenz: AAG AGT CTG TGC CCA AAT CAA) (SEQ ID NO:4);
   Caspase-9 ("siRNA CASP-9") (Hs_CASP9_7_HP Validierte siRNA, Katalog Nr SI02654610, Zielsequenz: CAG TGA CAT CTT TGT GTC CTA) (SEQ ID NO:5);
C: HP siRNA gegen humane NOX1 ("siRNA NOX1") ;
   Zielsequenz: CCG ACA AAT ACT ACT ACA CAA (SEQ ID NO:6)
D: siRNA gegen humane iNOS2 (siiNOS)
   Zielsequenz: CTG GGC CGT GCA AAC CTT CAA (SEQ ID NO:7)

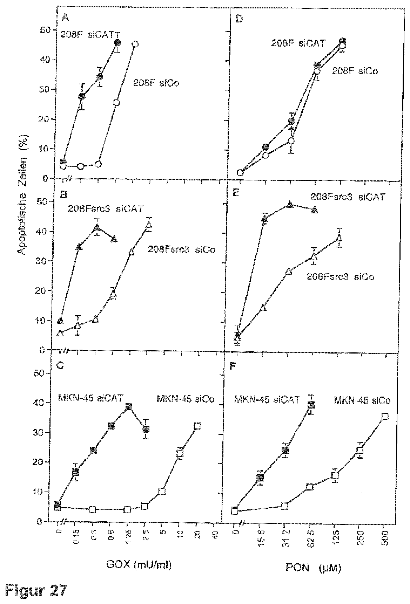

Für die in Fig. 27 vorgestellten Kontrolluntersuchungen wurden neben Kontroll siRNA auch siRNA gegen humane Katalase (für MKN-45-Zellen)
Hs_CAT_4_HP siRNA, Katalog. Nr. SI00027713
Zielsequenz: CCG GAT CTC ACT TGG CGG CAA (SEQ ID NO:8) und siRNA gegen murine Katalase (für 208F und 208Fsrc3-Zellen)
HP Mm_Cat_4_HP siRNA, Katalog. Nr. SI00941976
Zielsequenz: CCC AAT AGG AGA TAA ACT TAA (SEQ ID NO:9) eingesetzt.

Die Transfektionstechnik mithilfe der siRNAs wird bei Heinzelmann and Bauer, 2010 (a.a.O.) im Detail beschrieben. Die Transfektionseffizienz lag über 95 %. 24 Stunden nach Transfektion wurden Kontrolluntersuchungen auf die jeweiligen Genfunktionen durchgeführt, die sicherstellten, dass der "funktionelle Knockdown" vollständig war. Dies bedeutet, dass die spezifischen siRNAs die De-Novo-Synthese der analysierten Genprodukte wirkungsvoll unterbunden hatten und, dass der natürliche Abbau der vor siRNA-Gabe bestehenden Konzentration der Genprodukte bis unter die Nachweisgrenze erfolgt war.

Die humane Magenkarzinomlinie MKN-45 wurde in RPMI 1640 Medium, angereichert mit 10 % inaktiviertem Fetalen Bovinen Serum und 40 U/ml Penicillin, 50 µg/ml Streptomycin, 10 µg/ml Neomycin, 10 U/ml Moronal (antimykotisches Antibiotikum) und 280 µg/ml Glutamin gehalten. Die humane Neuroblastomlinie SHEP, sowie normale Rattenfibroblasten (208F) und deren durch das src-Onkogen transformierten Nachkommen (208Fsrc3) wurden in Eagle's Minimum Essential Medium (EMEM), angereichert mit 5 % inaktiviertem Fetalen Bovinen Serum und 40 U/ml Penicillin, 50 µg/ml Streptomycin, 10 µg/ml Neomycin, 10 U/ml Moronal und 280 µg/ml Glutamin gehalten. Details zu den Zelllinien und ihrer Kultur finden sich in den Arbeiten Heinzelmann and Bauer, 2010 bzw. Bechtel and Bauer, 2009.

### Beispiel 3: Standardansatz zur Analyse der autokrinen ROS-gesteuerten Apoptoseinduktion in Tumorzellen

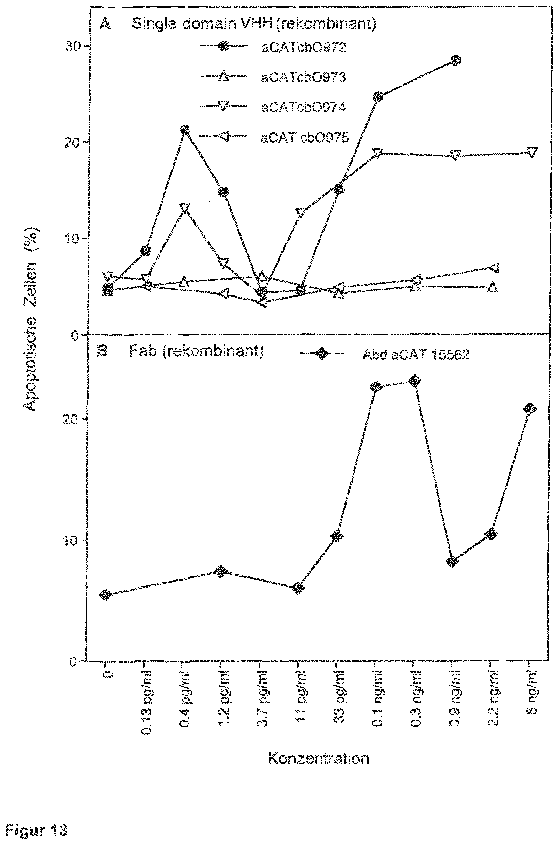

Die in Fig. 13-24 gezeigten Experimente wurden mit einem Standardansatz zur Apoptoseinduktion durchgeführt, der auf dem in Heinzelmann und Bauer 2010 ausgeführten Verfahren basiert. Die eingesetzten Tumorzellen wurden aus einer optimal wachsenden, halbdichten Kultur entnommen, zentrifugiert und in frischem Medium aufgenommen. Der Test erfolgte in 96-Loch Zellkulturplatten, mit 12 500 MKN-45-Zellen/100 µl Medium oder 10 000 SHEP-Zellen/100 µl Medium. MKN-45-Zellen wachsen in Suspension, SHEP-Zellen sind adhärent. Die Versuche mit SHEP-Zellen wurden begonnen sobald die Zellen angewachsen waren. Durch Zugabe steigender Konzentrationen von Single-Domain-VHH-Fragmenten, die entweder Katalase oder SOD inhibieren, wurde die Protektion der Tumorzellen vor ihrem eigenen ROS-Signaling außer Kraft gesetzt, so dass aufbauend auf der zelleigenen extrazellulären Superoxidanionenproduktion der NO/Peroxynitrit-Signalweg und der HOCI-Weg (bei MKN-45-Zellen) oder im Falle der SHEP-Zellen nur der NO/Peroxynitritweg einsetzen konnten, wenn die Zellen bei 37°C inkubiert wurden. Die Analyse der Signalwege erfolgte durch den Einsatz von Hemmstoffen der NADPH-Oxidase (AEBSF), dem HOCI-Fänger Taurin, dem Peroxynitritfänger FeTPPs und dem Singulettsauerstoff-Fänger Histidin. In einigen Experimenten wurden zum Vergleich statt der Single-Domain-VHH-Fragmente entweder rekombinante Fab-Fragmente oder monoklonale Antikörper eingesetzt. Dies ist in den jeweiligen Abbildungen gekennzeichnet.

Doppelansätze wurden nach den im Text genannten Zeiten mithilfe der Phasenkontrast-Umkehrmikroskopie auf den Prozentsatz apoptotischer Zellen untersucht. Dabei wurden die in Heinzelmann und Bauer 2010 beschriebenen und dokumentierten klassischen Apoptosekriterien wie Kernkondensation, Kernfragmentation und Membrane Blebbing verwendet. Pro Einzelansatz wurden mindestens 250 zufällig ausgewählte Zellen auf das Vorliegen von Apoptosemerkmalen untersucht.

Parallele Kontrolluntersuchungen, wie sie z. B. in der Arbeit Bauer et al. (Bauer G, Bereswill S, Aichele P and Glocker E. Helicobacter pylori protects oncogenically transformed cells from reactive oxygen species-mediated intercellular induction of apoptosis, Carcinogenesis 35: 1582-1591, 2014, Supplement) dokumentiert sind, stellten sicher, dass die angewandten morphologischen Kriterien mit Apoptosekriterien wie DNA-Fragmentierung (gemessen durch die TUNEL-Reaktion) oder Positivität für Annexin V-Bindung korrelierten.

### Beispiel 4: Spezifische Sensitivierung von Tumorzellen für Apoptose-auslösendes ROS-Signaling durch Single-Domain VHH-Fragmente gegen Katalase

Im Rahmen der vorliegenden Erfindung wurden viele Versuche durchgeführt, wobei die Versuchsergebnisse zusammengefasst in den Figuren 13 bis 30 dargestellt wurden.

Fig. 13 zeigt die spezifische Apoptoseinduktion in MKN-45 Magenkarzinomzellen durch Single-Domain-VHH-Fragmente gegen Katalase.

MKN-45 Zellen wurden unter Standardbedingungen für autokrine Apoptoseinduktion mit den angegebenen Konzentrationen von Single-Domain-VHH-Fragmenten, die an humane Katalase binden, diese aber nicht hemmen (aCATcb0973, aCATcb0975) und Single-Domain-VHH-Fragmenten, die an humane Katalase binden und diese hemmen (aCATcb0972; aCATcb9074) versetzt und für 3.5 Stunden bei 37 °C, 5 % CO₂ weiterinkubiert. Parallel wurden unter gleichen Bedingungen rekombinante Fab-Fragmente (bestehend aus leichter und schwerer Kette) eingesetzt, die gegen humane Katalase gerichtet sind und diese neutralisieren (Abd aCAT15562). Danach erfolgte die Bestimmung des Prozentsatzes apoptotischer Zellen (Doppelansätze) nach den oben aufgeführten klassischen Apoptosekriterien.

Die Fig. 13A zeigt, dass nur Single-Domain-VHH-Fragmente, die Katalase inhibieren, nicht aber solche, die an das Enzym binden ohne es zu inhibieren, Apoptose in den Tumorzellen auslösen. Dabei stellt sich die Wirkung in Form einer Optimumskurve dar, wie dies auch in der Arbeit von Heinzelmann and Bauer, 2010 für den Katalasehemmstoff 3-Aminotriazol beschrieben worden ist. Nach dem Abfall der Optimumskurve kommt es zu einem zweiten Anstieg der Apoptoseinduktion. Die Zugabe des aus leichten und schweren Ketten bestehenden rekombinanten Fab-Fragments Abd aCAT 15562 führt ebenfalls zur Apoptoseinduktion in Form einer Optimumskurve mit nachfolgendem zweiten Anstieg, doch wird im Vergleich zu den Katalase-inhibierenden Single-Domain-VHH-Fragmenten eine ca. 250-fach höhere molare Konzentration von Abd aCAT 15562 benötigt, um das Wirkungsoptimum zu erreichen. Dies belegt die überlegene Wirksamkeit der erfindungsgemäßen Konstrukte.

Die Zugabe von Single-Domain-VHH-Fragmenten und klassischen Fab-Fragmenten, die humane Katalase hemmen, führt in der Magenkarzinomzelllinie MKN-45 zur Induktion der Apoptose in Form einer Optimumskurve in Bezug auf die Konzentration der Antikörper. Die Spezifität des induzierten Prozesses wird dadurch belegt, dass Single-Domain-VHH-Fragmente, die an Katalase binden, diese aber nicht neutralisieren, zu keiner Apoptoseinduktion führen (Fig. 13). Es genügt für die Sensitivierung also nicht, dass Antikörper nur an die Katalase binden. Die spezifische Hemmung der Funktion der Katalase scheint für die Sensitivierung unerlässlich zu sein. Bemerkenswert und unerwartet ist der Befund, dass die Single-Domain-VHH-Fragmente eine weitaus stärkere Apoptose-auslösende Wirkung zeigen ais klassische Fab-Fragmente. Aufgrund des unterschiedlichen Aufbaus dieser beiden Gruppen von Fragmenten wäre zu erwarten gewesen, dass von den klassischen Fab-Fragmenten die doppelte Konzentration im Vergleich zu Single-Domain-VHH-Fragmenten einzusetzen gewesen wäre um eine gleiche Wirkung zu erzielen. Der Unterschied macht jedoch einen Faktor von ca. 500 (auf molarer Basis von 250) aus.

### Beispiel 5: Wirkung eines Single-Domain-VHH-Fragmentes, das Katalase bindet und Hemmung an Magenkarzinomzellen

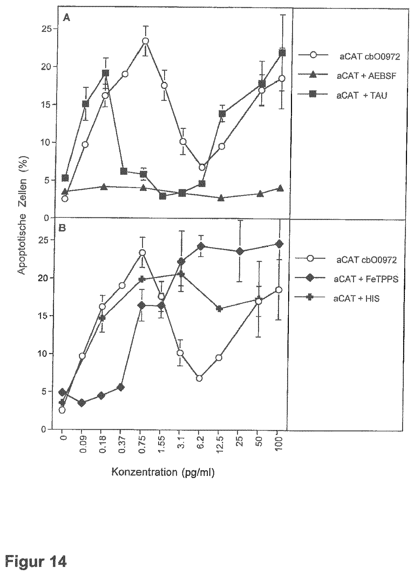

Fig. 14 zeigt, dass das Katalase-neutralisierende Single-Domain-VHH-Fragment aCATcb0972 in Magenkarzinomzellen der Linie MKN-45 spezifisches ROS-Signaling über den NO/Peroxynitrit- und HOCI-Weg induziert.

Zu Standardansätzen zur Induktion der Apoptose wurden die angegebenen Konzentrationen des Single-Domain-VHH-Fragments aCATcb0972 in Anwesenheit von 100 µM des NOX1-Inhibitors AEBSF, 50 mM des HOCI-Fängers Taurin (TAU), 25 µM des Peroxynitritfängers FeTPPS oder 2 mM des Singulettsauerstoff-Fängers Histidin (HIS) gegeben. Kontrollansätze wurden ohne Inhibitoren mitgeführt. Nach 3.5 Stunden bei 37°C, 5 % CO₂ wurden die Prozentsätze apoptotischer Zellen bestimmt.

Fig. 14 zeigt, dass das Single-Domain-VHH-Fragment aCATcb0972 (bindet an Katalase und hemmt diese) Apoptose in Form einer Optimumskurve und einem nachfolgenden zweiten Anstieg induziert. Dabei wird die Apoptoseinduktion im gesamten Konzentrationsbereich des Single-Domain-VHH-Fragments durch den NOX1-Inhibitor AEBSF gehemmt. Der HOCI-Fänger Taurin hemmt nicht im linken Konzentrationsbereich der Optimumskurve und führt dann jedoch im gesamten weiteren Bereich der Optimumskurve zu einer starken Hemmung der Apoptose. Der zweite Anstieg der Apoptose im Konzentrationsbereich ab 6.2 pg/ml des Single-Domain-VHH-Fragments wird durch Taurin nicht gehemmt. Der Peroxynitritfänger FeTPPS führt nur im linken Teil der Optimumskurve zu einer starken Hemmung und überführt dann die Optimumskurve der Apoptoseinduktion in eine Plateaukurve. Der Singulettsauerstoff-Fänger Histidin führt zu keiner Hemmung der Apoptose, überführt jedoch die Optimumskurve in eine Plateaukurve.

Die Fig. 14 belegt also, dass die Apoptose-auslösende Wirkung des Single-Domain-VHH-Fragments aCATcb0972 tatsächlich durch Reaktivierung des ROS-Signaling bedingt ist, da bei allen eingesetzten Konzentrationen eine vollständige Hemmung der Apoptoseinduktion erfolgt, wenn die Superoxidanionen-Produktion durch AEBSF gehemmt wird. Das in Fig. 14 belegte Ergebnis zeigt außerdem, dass der Optimumsbereich der Apoptoseinduktion (0-6.2 pg/ml) durch eine Aufeinanderfolge des NO/Peroxynitritwegs und des HOCI-Wegs gekennzeichnet ist. Im Bereich von 0.09 und 0.18 pg/ml aCATcb0972 erfolgt Hemmung durch den Peroxynitritfänger FeTPPS, aber keine Hemmung durch den HOCI-Fänger Taurin, was den Ablauf des NO/Peroxynitritwegs anzeigt. Ab 0.75 pg/ml des Fab-Fragments erfolgt keine wesentliche Hemmung durch FeTTPS, jedoch sehr starke Hemmung durch Taurin, was das Ablaufen des HOCI-Wegs beweist. Bei 0.37 pg/ml überlappen sich erwartungsgemäß beide Signalwege. Der Wiederanstieg der Apoptoseinduktion bei höheren Konzentrationen des Single-Domain-VHH-Fragmentes hängt
i) von der Produktion von Superoxidanionen,
ii) vom Grad der Katalasehemmung ab und wird weder durch den HOCI-Fänger, noch den Peroxynitritfänger beeinträchtigt.

Es handelt sich demnach um einen reinen Effekt von H₂O₂, und nicht um einen der spezifischen ROS-Signalwege.

Das Überführen der Optimumskurve der Apoptoseinduktion in eine Plateaukurve durch den Singulettsauerstoff-Fänger Histidin und den Peroxynitritfänger FeTPPS weist darauf hin, dass bei Konzentrationen des Single-Domain-VHH-Fragmentes aCATcb0872 > 0.75 pg/ml auch Singulettsauerstoff eine Rolle zu spielen scheint. Singulettsauerstoff kann durch die Reaktion von H₂O₂ mit Peroxynitrit entstehen. Von Singulettsauerstoff ist auch bekannt, dass er Katalase inaktivieren kann. Die dadurch bewirkte erhöhte Verfügbarkeit von H₂O₂ kann dann die in Fig. 1 gezeigten Nebenreaktionen⑧ und ⑨ auslösen, wodurch HOCI verbraucht oder weniger HOCI synthetisiert wird. Dadurch wird der Abfall der Apoptoseinduktion auf der rechten Seite der Optimumskurve bewirkt.

### Beispiel 6 Spezifische Sensitivierung von Tumorzellen für Apoptose-auslösendes ROS-Signaling durch Single-Domain-VHH-Fragmente gegen SOD

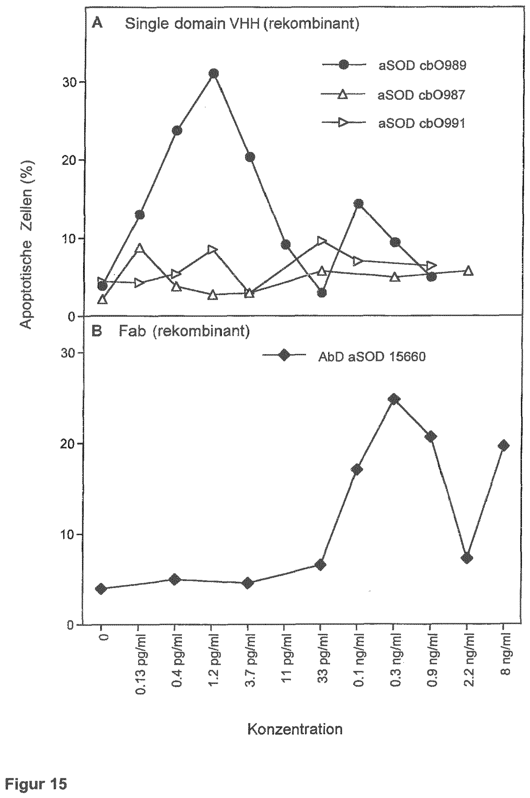

Die spezifische Apoptoseinduktion in MKN-45 Magenkarzinomzellen durch Single-Domain-VHH-Fragmente gegen SOD wird in Fig. 15 gezeigt. Der prozentuale Anteil von apoptotischen Zellen ist an der X-Achse aufgetragen.

MKN-45 Zellen wurden unter Standardbedingungen für autokrine Apoptoseinduktion mit den angegebenen Konzentrationen von Single-Domain-VHH-Fragmenten, die an humane SOD1 binden, diese aber nicht hemmen (aSODcb0987, aSODcb0991) und Single-Domain-VHH-Fragmenten, die an humane SOD binden und diese hemmen (aSODcb0989) versetzt und für 3.5 Stunden bei 37 °C, 5 % CO₂ weiter inkubiert. Parallel wurden unter gleichen Bedingungen rekombinante Fab-Fragmente (bestehend aus leichter und schwerer Kette) eingesetzt, die gegen humane Katalase gerichtet sind und diese neutralisieren (Abd aSOD 15660). Danach erfolgte die Bestimmung des Prozentsatzes apoptotischer Zellen (Doppelansätze) nach den oben aufgeführten klassischen Apoptosekriterien.

Die Fig. 15A zeigt, dass Single-Domain-VHH-Fragmente, die SOD inhibieren, nicht aber solche, die nur an das Enzym binden, Apoptose in den Tumorzellen auslösen. Dabei stellt sich die Wirkung in Form einer breiten Optimumskurve dar. Eine Optimumskurve der Apoptoseinduktion wird auch durch das klassische Fab-Fragment AbD aSOD 15660 erzielt (Fig. 15B), allerdings ist dazu eine im Vergleich zum Single-Domain-VHH-Fragment 250-fach höhere Konzentration notwendig, um die gleiche Wirkung zu erzielen.

Die Fig. 15 zeigt also, dass es für die Sensitivierung nicht genügt, dass Antikörper an die SOD binden. Die spezifische Hemmung der Funktion der SOD scheint für die Sensitivierung unerlässlich zu sein. Bemerkenswert ist, dass die gezielte Hemmung der membranständigen SOD ausreicht, um das zur Apoptose führende ROS-Signaling zu reaktivieren. Bemerkenswert und unerwartet ist der Befund, dass neutralisierende Single-Domain-VHH-Fragmente eine weitaus stärkere Apoptose-auslösende Wirkung zeigen als klassische neutralisierende Fab-Fragmente. Aufgrund des unterschiedlichen Aufbaus dieser beiden Gruppen von Fragmenten wäre zu erwarten gewesen, dass von den klassischen Fabs die doppelte Konzentration im Vergleich zu Single-Domain-VHH-Fragmenten einzusetzen gewesen wäre um eine gleiche Wirkung zu erzielen. Der Unterschied beträgt jedoch einen Faktor von ca. 250.

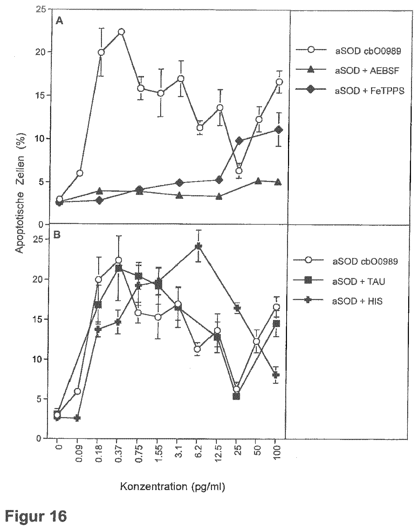

Fig. 16 zeigt, dass das SOD-neutralisierende Single-Domain-VHH-Fragment aSODcb0989 in Magenkarzinomzellen der Linie MKN-45 spezifisches ROS-Signaling ausschließlich über den NO/Peroxynitrit-Weg induziert.

Zu Standardansätzen zur Induktion der Apoptose wurden die angegebenen Konzentrationen des Single-Domain-VHH-Fragments aSODcb0989 in Anwesenheit von 100 µM des NOX1-Inhibitors AEBSF, 25 µM des Peroxynitritfängers FeTPPS, 50 mM des HOCI-Fängers Taurin (TAU) oder 2 mM des Singulettsauerstoff-Fängers Histidin (HIS) gegeben. Kontrollansätze wurden ohne Inhibitoren mitgeführt. Nach 3.5 Stunden bei 37°C, 5 % CO₂ wurden die Prozentsätze apoptotischer Zellen bestimmt.

Die Fig. 16 zeigt, dass SOD-neutralisierende Single-Domain-VHH-Fragmente in Magenkarzinomzellen der Linie MKN-45 Apoptose in Form einer breiten Optimumskurve induzieren, auf den ein angedeuteter zweiter Anstieg erfolgt. Die Apoptoseinduktion wird im gesamten Konzentrationsbereich durch AEBSF gehemmt. Der gesamte Optimumsbereich wird durch FeTPPS, nicht aber durch Taurin gehemmt. Histidin führt zu einer erkennbaren teilweisen Aufhebung des Abfalls der rechten Flanke der Optimumskurve.

Die Fig. 16 belegt also, dass durch aSODcb0989 induzierte Apoptose in den Tumorzellen zwar durchgängig über einen Superoxidanionen-abhängigen Prozess ausgelöst wird, dass es sich dabei aber (im Gegensatz zu aCATcb0972) ausschließlich um den NO/Peroxynitritweg handelt und HOCI-Signaling keinen erkennbaren Einfluss hat. Dies wird durch die vollständige Hemmung mittels FeTPPS und das Ausbleiben einer Hemmung durch Taurin belegt. Dieses Ergebnis weist darauf hin, dass nach Wegfall der SOD-Wirkung offensichtlich nicht ausreichend H₂O₂ für den Ablauf des HOCI-Wegs zur Verfügung steht. Da der NO/Peroxynitritweg sehr effizient durch membranständige Katalase gehemmt wird, lässt das in Fig. 16 gezeigte Ergebnis den Schluss zu, dass die Hemmung der SOD durch das Single-Domain-VHH-Fragment auch zu einer indirekten Hemmung der Katalase geführt haben muss. Die unterschiedliche Qualität der durch aSODcb0989 und aCATcb0972 reaktivierten Signalwege erlaubt den Ausschluss der theoretischen Annahme, dass es sich bei der Wirkung von aSODcb0989 um eine Kreuzreaktion mit Katalase handeln könnte und bestätigt dadurch ebenfalls die Spezifität der Wirkung von aSODcb0989.

### Beispiel 7: Verhältnis von Single-Domain-VHH-Fragmenten zu Zielzellen

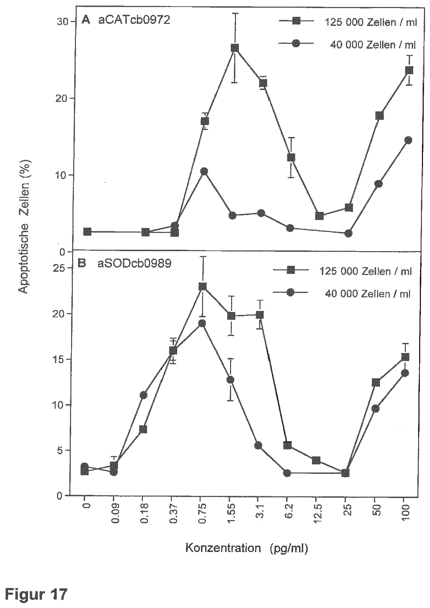

Fig. 17 zeigt, dass Single-Domain-VHH-Fragmente gegen Katalase bzw. SOD unterschiedliche Ansprüche an die Dichte der Zielzellen stellen.

Ansätze zur Induktion der Apoptose wurden neben der Standard-Zelldichte (12 500 Zellen/100 µl) auch mit einer niedrigeren Zelldichte (4 000 Zellen/100 µl) und steigenden Konzentrationen der Single-Domain-VHH-Fragmente aCATcb0972 bzw. aSODcb0989 angesetzt und für vier Stunden bei 37°C, 5 % CO₂ inkubiert. Danach wurden die Prozentsätze apoptotischer Zellen bestimmt.

Fig. 17 zeigt, dass die Apoptoseinduktion durch die gegen Katalase gerichteten Single-Domain-VHH-Fragmente nur bei der höheren Dichte in optimaler Weise abläuft, während sich bei Anwendung der gegen SOD gerichteten Single-Domain-VHH-Fragmente eine weitaus geringere Abschwächung der Wirkung einstellt, wenn die Zielzellen in niedrigerer Dichte vorliegen. Dieser Unterschied belegt die unterschiedliche Qualität der reaktivierten Signalwege und steht insbesondere damit in Einklang, dass Hemmung der SOD vorzugsweise den NO/Peroxynitritweg reaktiviert, da dieser nicht in dem Maße an eine hohe Zelldichte gebunden ist wie der HOCI-Signalweg.

### Beispiel 8: Synergistischer Effekt von Single-Domain-VHH-Fragmenten gegen Katalase und SOD

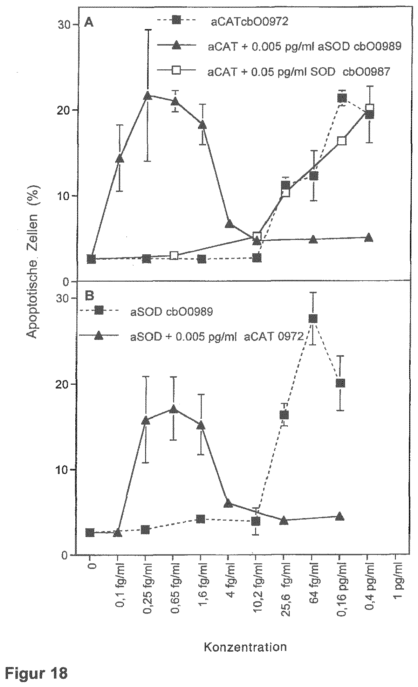

Fig. 18 zeigt einen bemerkenswerten synergistischen Effekt bei gleichzeitiger Anwendung von Single-Domain-VHH-Fragmenten gegen Katalase und SOD.

Standardansätze zur Apoptoseinduktion mit MKN-45 Zellen wurden mit steigenden Konzentrationen der Katalase neutralisierenden Single-Domain-VHH-Fragmente aCATcb0972 allein bzw. in Kombination mit 0.005 pg/ml des SOD-neutralisierenden Single-Domain-VHH-Fragments aSODcb0989 oder des SOD-bindenden, aber nicht neutralisierenden Single-Domain-VHH-Fragments aSODcb0987 versetzt (A). Im komplementären Experiment (B) wurden steigende Konzentrationen von aSODcb0989 allein oder in Kombination mit 0.005 pg/ml des Katalase neutralisierenden Single-Domain-VHH-Fragments aCATcb0972 versetzt. Alle Ansätze wurden für 3.5 Stunden bei 37 °C, 5 % CO₂ inkubiert. Danach wurden die Prozentsätze apoptotischer Zellen bestimmt.

Fig. 18 zeigt, dass sich die bereits in den Vorexperimenten gemessenen konzentrationsabhängigen Apoptose-auslösenden Effekte sowohl durch aCATcb0972 als auch aSODcb0989 bestätigen lassen. Die Kombination mit einer geringen, für sich alleine nicht zur Apoptoseinduktion ausreichenden Konzentration des jeweils komplementären Single-Domain-VHH-Fragments (also aSOD zu steigenden Konzentrationen aCAT und aCAT zu steigenden Konzentrationen aSOD) führte zu einem bemerkenswerten synergistischen Effekt bei der Apoptoseinduktion. Die Spezifität dieses Synergieeffektes wird dadurch belegt, dass er sich bei Gabe eines SOD-bindenden, aber nicht neutralisierenden Single-Domain-VHH-Fragments (Fig. 18 A) nicht einstellte.

Fig. 18 zeigt also, dass die Kombination von Single-Domain-VHH-Fragmenten gegen Katalase bzw. SOD zu einem sehr starken Synergieeffekt führt. Bei optimaler Nutzung dieses Effektes könnten die benötigten Konzentrationen an Single-Domain-VHH-Fragmenten drastisch abgesenkt werden.

### Beispiel 9: Synergistischer Effekt mit Chemotherapeutikum

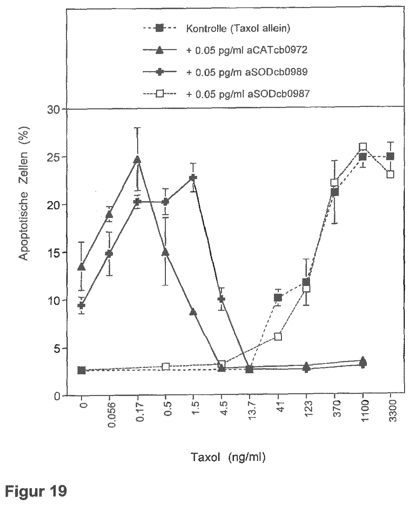

Fig. 19 zeigt, dass Katalase bzw. SOD neutralisierende Single-Domain-VHH-Fragmente einen starken synergistischen Effekt mit dem etablierten Chemotherapeutikum Taxol bewirken.

Standardansätze zur Apoptoseinduktion mit MKN-45 Zellen wurden mit steigenden Konzentrationen Taxol alleine oder in Kombination mit Katalase neutralisierendem aCATcb0972, SOD-neutralisierendem aSODcb0989 und SOD-bindendem, aber nicht neutralisierendem aSODcb0987 versetzt und für 4 Stunden bei 37 °C, 5 % CO₂ inkubiert. Danach wurden die Prozentsätze apoptotischer Zellen bestimmt.

Fig. 19 zeigt, dass Taxol konzentrationsabhängig Apoptose in den Tumorzellen auslöst.

Die optimale Wirkung wurde dabei im Konzentrationsbereich von 1100 ng/ml festgestellt. Diese Wirkung war zu erwarten. Bei Kombination mit Single-Domain-VHH-Fragmenten, die entweder Katalase oder SOD inhibieren konnten, verschob sich überraschender Weise die Taxol-abhängige Optimumskurve drastisch in einen niedrigeren Konzentrationsbereich. Das Optimum der Wirkung lag nun zwischen 0.17 ng/ml und 0.5 ng/ml. Diese beeindruckende Verschiebung der notwendigen Konzentration auf weniger als ein Tausendstel konnte nur durch Single-Domain-VHH-Fragmente erzielt werden, die die jeweilige Zielstruktur (Katalase oder SOD) auch neutralisierten, während eine bloße Bindung keinerlei Verstärkungseffekt hervorrief.

Dieser in Fig. 19 gezeigte Effekt ist zunächst deshalb hochinteressant, weil er eine bisher nicht bekannte funktionelle Verbindung zwischen der Wirkung von Taxol auf Tumorzellen und ROS-Signaling belegt. Das Auftreten eines Synergieeffektes mit Single-Domain-VHH-Fragmenten gegen SOD bzw. Katalase bietet die Möglichkeit, durch geeignete Kombination der Wirkstoffe insgesamt Ressourcen zu schonen, dadurch eine Kostensenkung der Therapie zu erzielen und eröffnet darüber hinaus die Chance, die durch Taxol bedingten Nebenwirkungen zu senken oder sogar zu vermeiden und mögliche Nebenwirkungen der Single-Domain-VHH-Fragmente zu vermeiden.

### Beispiel 10: Spezifische Sensitivierung von Tumorzellen für Apoptose-auslösendes ROS-Signaling durch Hybridmoleküle aus Katalase- bzw. SOD-neutralisierenden Single-Domain-VHH-Fragmenten

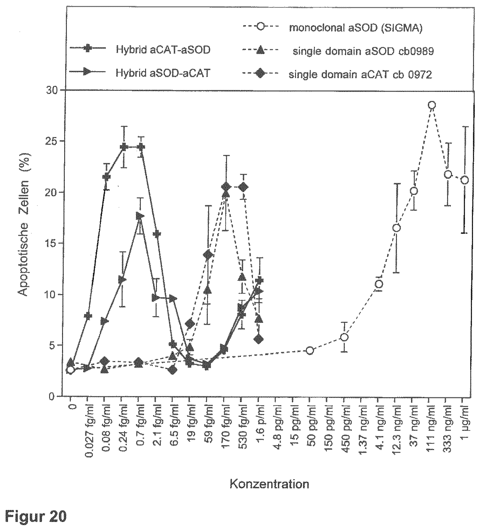

Fig. 20 zeigt, dass sich der Synergieeffekt zwischen Single-Domain-VHH-Fragmenten aCAT und aSOD in einem Hybridmolekül bündeln lässt.

Standardansätze zur Apoptoseinduktion mit MKN-45 Zellen wurden mit steigenden Konzentrationen von Katalase neutralisierendem aCATcb0972, SOD-neutralisierendem aSODcb0989, den Hybridmolekülen aus aCATcb0972 und aSODcb0989 in den zwei möglichen Anordnungen, sowie als Kontrolle einem neutralisierenden konventionellen monoklonalem Antikörper gegen Katalase (Sigma) versetzt für 3.5 Stunden bei 37 °C, 5 % CO₂ inkubiert. Danach wurden die Prozentsätze apoptotischer Zellen bestimmt.

Fig. 20 zeigt, dass sich der bei Kombination der einzeln zugegebenen Single-Domain-VHH-Fragmente aCATcb0972 und aSODcb0989 beobachtete Synergieeffekt tatsächlich in Hybridmolekülen bündeln lässt. Dabei schien die Anordnung aCATaSOD der umgekehrten Anordnung graduell überlegen zu sein. Während die optimale Wirkung der einzeln applizierten Single-Domain-VHH-Fragmente im Konzentrationsbereich um 170 fg/ml lag, wurde die optimale Wirkung durch die Hybridmoleküle bereits bei 0.24 fg/ml erzielt. Diesen bemerkenswerten Aktivitäten steht eine dramatisch geringere Wirksamkeit des konventionellen monoklonalen Antikörpers entgegen, der seine optimale Wirkung bei 111 ng/ml entfaltet. In die Abbildung wurden die Daten für klassische rekombinante Fab-Fragmente, die SOD bzw. Katalase neutralisieren, aus Gründen der Übersichtlichkeit nicht aufgenommen. Ihre optimale Wirksamkeit liegt im Bereich von 0.1-0.3 ng/ml.

Fig. 20 bestätigt also, dass
i) die Wirksamkeit von Single-Domain-VHH-Fragmenten unerwarteter Weise weitaus größer ist, als die konventioneller monoklonaler Antikörper oder konventioneller rekombinanter Fab-Fragmente und
ii) dass sich eine weitere sehr deutliche Steigerung der Wirksamkeit erzielen lässt, wenn Hybridmoleküle aus Single-Domain-VHH-Fragmenten generiert werden.

### Beispiel 11:

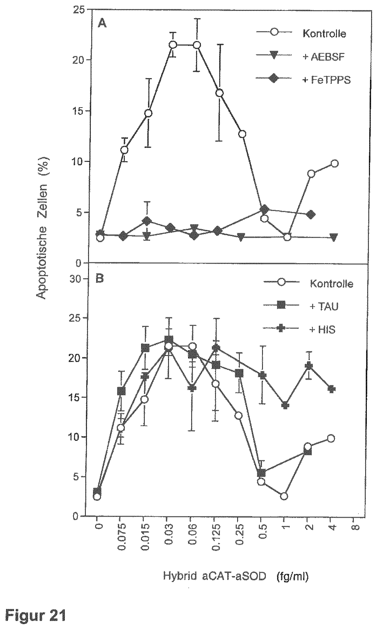

Fig. 21 zeigt, dass das Hybridmolekül aus den Single-Domain-VHH-Fragmenten aCATcb0972 und aSODcb0989 in Magenkarzinomzellen der Linie MKN-45 spezifisches ROS-Signaling ausschließlich über den NO/Peroxynitrit-Weg induziert.

Zu Standardansätzen zur Induktion der Apoptose wurden die angegebenen Konzentrationen des Hybridmoleküls aCATaSOD in Anwesenheit von 100 µM des NOX1-Inhibitors AEBSF, 25 µM des Peroxynitritfängers FeTPPS, 50 mM des HOCI-Fängers Taurin (TAU) oder 2 mM des Singulettsauerstoff-Fängers Histidin (HIS) gegeben. Kontrollansätze wurden ohne Inhibitoren mitgeführt. Nach 3 Stunden bei 37 °C, 5 % CO₂ wurden die Prozentsätze apoptotischer Zellen bestimmt.

Fig. 21 bestätigt, dass das Hybridmolekül aCATaSOD in den Tumorzellen Apoptose über die Reaktivierung des ROS-Signaling induziert, denn der Prozess wird durch Hemmung der Superoxidanionensynthese mittels AEBSF vollständig verhindert. Weiterhin ist erkennbar, dass es sich bei der durch das Hybridmolekül reaktivierten Apoptoseinduktion ausschließlich um den NO/Peroxynitritweg handelt, da eine vollständige Hemmung durch FeTPPS, aber keine Hemmung durch Taurin erfolgt. Der Singulettsauerstoff-Fänger zeigt wiederum eine abschwächende Wirkung auf den rechtsseitigen Abfall der Optimumskurve. Die Dominanz des NO/Peroxynitritwegs war unerwartet und aus den Vorergebnissen nicht vorhersagbar.

### Beispiel 12:

Fig. 22 bestätigt mittels siRNA-begründeter Analyse die Spezifität des durch das Hybridmolekül ausgelösten, zur Apoptose führenden ROS-Signaling.

MKN-45 Zellen wurden mit 24 nM siRNA transfiziert, die gegen NOX1, iNOS2, Caspase-9, FAS-Rezeptor oder Caspase-8 gerichtet war. Kontrollansätze wurden mit irrelevanter Kontroll-siRNA transfiziert. Nach 24 Stunden bei 37 °C wurden die Zellen in frischem Medium aufgenommen und mit den angegebenen Konzentrationen des Hybridmoleküls aCATaSOD versetzt. Die Bestimmung des Prozentsatzes der apoptotischen Zellen erfolgte nach vier Stunden.

Fig. 22 zeigt, dass die durch das Hybridmolekül aCATaSOD bewirkte Apoptoseinduktion in Form einer Optimumskurve nur dann ablaufen kann, wenn intakte NADPH Oxidase (NOX1), NO Synthase (iNOS2) und die für den Ablauf der Apoptose über den mitochondrialen Weg essentielle Caspase-9 zur Verfügung stehen. Der siRNA-vermittelte Knockdown dieser Enzyme verhindert jeweils die durch das Hybridmolekül ausgelöste Apoptoseinduktion vollständig und beweist daher das zugrunde liegende ROS Signaling über den NO/Peroxynitritweg mit nachfolgendem mitochondrialen Apoptosesignaling. Die Apoptoseinduktion durch das Hybridmolekül bedarf hingegen nicht des FAS-Rezeptors und dessen nachgeschalteter Caspase-8, was die ausschließliche Wirkung des mitochondrialen Apoptosewegs unterstreicht und ausschließt, dass der Apoptoseweg über den Todesrezeptor APO/FAS für die Apoptoseinduktion unter den gewählten Bedingungen eine Rolle spielt. Allerdings verhindert das Ausschalten des FAS-Rezeptors und der Caspase-8 den supraoptimalen Abfall der Apoptoseinduktions-Kurve. Dies ist durch die Beteiligung von FAS-Rezeptor und Caspase-8 and Singulettsauerstoff-bedingten Prozessen erklärbar, wie in Bauer, 2012 ausgeführt wird und deckt sich mit dem in Fig. 21 für Histidin erhobenen Befund.

Aus den Figuren 13, 15 und 20 wurden die Konzentrationen der Antikörper und Single-Domain-VHH-Fragmente bestimmt, die für optimale Apoptoseinduktion in den Tumorzellen notwendig waren (Tabelle 1). Diese Zusammenstellung verdeutlicht die überlegene Wirksamkeit der Single-Domain-VHH-Fragmente im Vergleich zu klassischen rekombinanten Fab-Fragmenten und zu monoklonalen Antikörpern. Tabelle 2 unterstreicht den beeindruckenden Synergieeffekt, der sich durch Verwendung der Hybridmoleküle erreichen lässt. Die überlegene Wirkung der Single-Domain-VHH-Fragmente gegenüber klassischen rekombinanten Fab-Fragmenten war nicht vorhersehbar und daher unerwartet. Aufgrund des bisherigen Kenntnisstandes war vielmehr zu erwarten, dass neutralisierende klassische Fab-Fragmente und neutralisierende Single-Domain-VHH-Fragmente bei Betrachtung der molaren Konzentration den gleichen Effekt auf die Apoptoseinduktion erreichen sollten, bei Betrachtung der Konzentrationen (pg/ml) sollte damit nur ein Unterschied um den Faktor 2 erreicht werden. Dieser nicht vorhersagbare dramatische Unterschied in der Wirksamkeit ist überraschend. Dieser Effekt eignet sich bestens für die therapeutische Anwendung.

**Tabelle 1:**

| **Antikörper** | **optimale Konz.** | **Relation zur opt. Konz. Single Domain Fab** | **Relation (molare Basis)** |
|---|---|---|---|
| aCAT cb0972 | 0.4 pg/ml | | |
| (Single domain VHH) | | | |
| AbD aCAT 15562 | 0.2 ng/ml | 500 | 250 |
| (rekomb. Fab klassisch) | | | |
| Monoclonal aCAT | 111 ng/ml | 277 500 | 46 250 |
| (Sigma) | | | |
| aSOD cb0989 | 1.2 pg/ml | | |
| (Single domain VHH) | | | |
| AbD aSOD 15660 | 0.3 ng/ml | 250 | 125 |

**Tabelle 2**

| **Antikörper** | **optimale Konz.** | **Relation zur opt. Konz. Hybrid Fab** | **Relation (molare Basis)** |
|---|---|---|---|
| aCATaSOD | | | |
| (Hybrid Single Dom. VHH) | 0.24 fg/ml | | |
| aCAT cb0972 | 0.17 pg/ml | 708 | 1416 |
| (Single domain VHH) | | | |
| Monoclonal aCAT | 111 ng/ml | 4.6 x 10⁸ | 1.54 x10⁸ |
| (Sigma) | | | |
| aSOD cb0989 | 0.35 pg/ml | 1458 | 2916 |
| (Single domain VHH) | | | |

Die Werte wurden den Figuren 13, 15 und 20 entnommen. Zunächst wurde bestimmt, um wievielfach höher die Konzentration von herkömmlichen rekombinanten Fab-Fragmenten und monoklonalen Antikörpern sein muss, um den gleichen Effekt zu erzielen wie mit rekombinanten Single-Domain-VHH-Fragmenten. Durch Einbeziehen der molaren Massen der verschiedenen Fab-Fragmente und Antikörper wurde dann die Relation auf der Basis von Molaritäten bestimmt. Bei dieser Korrektur wurde die gültige Annahme eingesetzt, dass klassische Fab-Fragmente ein Drittel der molaren Masse eines kompletten IgG-Moleküls ausmachen und dass die molare Masse eines Single-Domain-VHH-Fragmentes in etwa die Hälfte der molaren Masse eines klassischen Fab-Fragmentes ausmacht.

### Beispiel 13: Wirkung von Single-Domain-VHH-Fragmenten mit Katalase- bzw. SOD neutralisierender Wirkung auf humane Tumorzellen, die nur zur Etablierung des NO/Peroxynitritweges befähigt sind.

Während die in den bisherigen Beispielen verwendete humane Magenkarzinomlinie MKN-45 dadurch gekennzeichnet ist, dass sie das gesamte Spektrum des bekannten interzellulären ROS-Signaling (HOCI und NO/Peroxynitritweg als Hauptwege, Nitrylchloridweg als Nebenweg) ausprägen kann, wenn ihre membranständige Katalase gehemmt wird, zeigt sich bei einer Reihe anderer humaner Tumorzell-Linien eine Beschränkung auf den NO/Peroxynitritweg (Heinzelmann and Bauer, 2010; Bauer, 2012). In den bisherigen Untersuchungen wurde dabei festgestellt, dass ein bestimmter Tumortyp jeweils ein einheitliches ROS-Signalsystem zeigt. Begrenzung auf das NO/Peroxynitrit-Signaling wurde von uns bisher bei Neuroblastomen, Ewing-Sarcomen, Mammakarzinomen, Ovarialkarzinomene und kleinzelligen Lungenkarzinomen beobachtet.

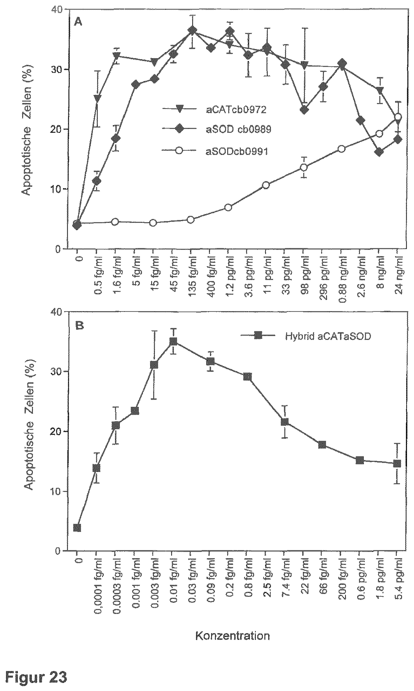

Fig. 23 zeigt, dass Single-Domain-VHH-Fragmente auch Apoptose in Tumorzellen reaktivieren können, die ausschließlich NO/Peroxynitritsignaling ausbilden können.

10 000 Zellen der humanen Neuroblastomlinie SHEP pro 100 µl Medium wurden mit den angegebenen Konzentrationen des Katalase-neutralisierenden Single-Domain-VHH-Fragments aCATcb0972, des SOD-neutralisierenden Single-Domain-VHH-Fragments aSOD0989, des SOD-bindenden, aber nicht neutralisierenden Single-Domain-VHH-Fragments aSODcb991 und des Hybridmoleküls aCATaSOD versetzt und für 5 Stunden bei 37 °C, 5 % CO₂ inkubiert, bevor die Prozentsätze apoptotischer Zellen bestimmt wurden.

Fig. 23 zeigt, dass die Katalase- und SOD-neutralisierenden Single-Domain-VHH-Fragmente, sowie das Hybridmolekül aCATaSOD in der Neuroblastomlinie SHEP konzentrationsabhängig Apoptose auslösen können. Dabei erwies sich das Hybridmoleküi erwartungsgemäß als deutlich wirksamer als die Single-Domain-VHH-Fragmente, die jeweils nur gegen eine Zielstruktur gerichtet sind. Das SOD-bindende, aber nicht neutralisierende Single-Domain-VHH-Fragment aSODcb0991 erreicht bei hohen Konzentrationen ebenfalls eine Apoptose-auslösende Wirkung, doch ist dafür eine 10⁶-fach höhere Konzentration notwendig als für die neutralisierenden Single-Domain-VHH-Fragmente.

Fig. 23 belegt also, dass auch Zell-Linien, die nach Katalasehemmung ausschließlich zu NO/Peroxynitritsignaling befähigt sind, durch Single-Domain-VHH-Fragmente, die gegen Katalase oder SOD gerichtet sind, in die Apoptose getrieben werden können. Dabei stellt sich ebenfalls der Synergieeffekt zwischen aCAT und aSOD ein. Bemerkenswert ist die bei dieser Zelllinie beobachtete Konzentrations-Wirkungskurve, die im Vergleich zur Zelllinie MKN-45 nur einen sehr flachen, langanhaltenden supraoptimalen Abstieg an der rechten Flanke aufweist.

Die schwache, aber signifikante Apoptoseinduktion, die durch das Single-Domain-VHH-Fragment aSODcb0991, das nur binden, aber nicht neutralisieren kann, erreicht wird, ist am besten dadurch zu erklären, dass nach Bindung des Single-Domain-VHH-Fragments eine Internalisierung der SOD erfolgt und daher deren Konzentration auf der Oberfläche verringert wird, was eine zur Hemmung analoge Wirkung nach sich ziehen sollte.

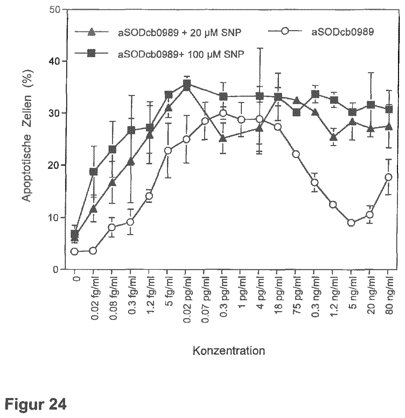

Fig. 24 zeigt, dass die Wirkung von aSODcb0989 auf SHEP-Zellen weiter optimiert werden kann, wenn zusätzlich der NO-Donor SNP eingesetzt wird.

10 000 Zellen der humanen Neuroblastomlinie SHEP pro 100 µl Medium wurden mit den angegebenen Konzentrationen des SOD-neutralisierenden Single-Domain-VHH-Fragments aSOD0989 versetzt. Parallele Ansätze erhielten 20 µM oder 100 µM des NO Donors Sodium Nitroprussid oder wurden ohne weiteren Zusatz inkubiert. Nach 5 Stunden bei 37°C, 5 % CO₂ wurden die Prozentsätze apoptotischer Zellen bestimmt.

Fig. 24 zeigt, dass die zusätzliche Gabe des NO-Donors (der für sich alleine keine Apoptose induzieren kann) sowohl zu einer Sensitivierung bezüglich der Reaktivierung der Apoptoseinduktion führt, als auch dem supraoptimalen rechtsseitigen Abfall der Optimumskurve wirkungsvoll entgegen wirkt. Daraus sollten sich Modulierungsverfahren etablieren lassen, die die Optimumskurve der Apoptoseinduktion in eine Plateaukurve transformieren, woraus sich eine größere Sicherheit der therapeutischen Anwendung ergeben sollte.

### Beispiel 14: Single-Domain-VHH-Fragmente inhibieren ausschließlich membranständige Katalase (die für Tumorzellen charakteristisch und essentiell ist) und erreichen intrazelluläre Katalase (die auch in Normalzellen eine Rolle spielt) nicht.

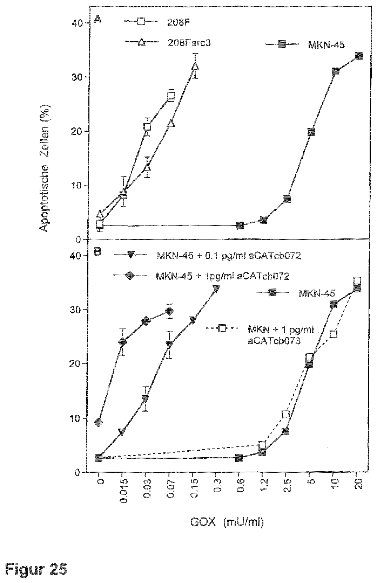

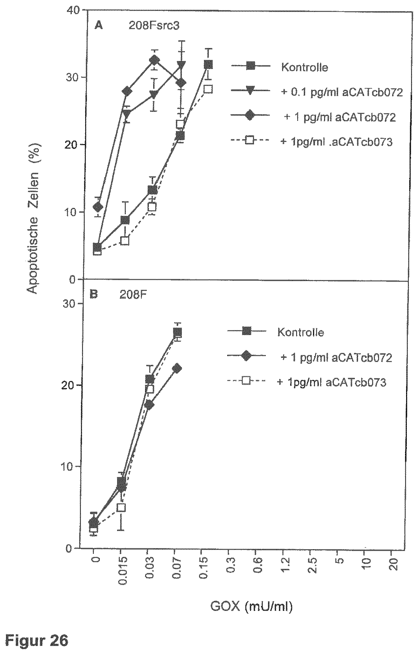

Fig. 25 und 26 zeigen, dass Katalase-neutralisierende Single-Domain-VHH-Fragmente nur die membranständige Katalase von Tumorzellen neutralisieren und die intrazelluläre Katalase von Normalzellen nicht beeinflussen können.

6000 nicht transformierte 208F Zellen, transformierte 208Fsrc3-Zellen bzw. MKN-45 Tumorzellen wurden jeweils in 100 µl Medium eingesät und mit 0.1 oder 1 pg/ml Katalaseneutralisierendem aCATcb0972 oder nur Katalase-bindendem aCATcb0973 versetzt. Kontrollansätze blieben ohne Single-Domain-VHH-Fragmente. Anschließend wurden die angezeigten Konzentrationen Glucoseoxidase zugegeben und die Apoptoseinduktion wurde nach 1.5 Stunden gemessen. Glucoseoxidase (GOX) generiert H₂O₂, welches zellgängig ist und daher sowohl durch intrazelluläre als auch membranständige Katalase abgebaut werden kann. In ausreichender Konzentration induziert H₂O₂ Apoptose ohne Selektivität in Bezug auf den malignen Status von Zellen (Ivanovas et al., Selective and nonselective apoptosis induction in transformed and nontransformed fibroblasts by exogenous reactive oxygen and nitrogen species. Anticancer Research, Anticancer Res. 22:841-856, 2002).

Fig. 25A zeigt, dass die Tumorzelllinie MKN-45 weitaus besser gegen H₂O₂ geschützt ist als die Normalzellen und die transformierte Zelllinie. In Anwesenheit von Katalase-neutralisierenden Single-Domain-VHH-Fragmenten werden die Tumorzellen sehr deutlich für die Wirkung des H₂O₂ sensitiviert, während Single-Domain-VHH-Fragmente, die nur an Katalase binden, zu keiner Sensitivierung führten. Abbildung 26 zeigt, dass die neutralisierenden Single-Domain-VHH-Fragmente eine sensitivierende Wirkung auf die transformierte Linie bewirkten, die Normalzellen jedoch nicht beeinflussen konnten.

Fig. 25 und 26 belegt die spezifische Wirkung der Single-Domain-VHH-Fragmente auf die malignen Zellen, während die Normalzellen nicht beeinflusst werden. Dabei war die größte Wirkung bei den Tumorzellen zu beobachten, da diese vor allem durch membranständige Katalase geschützt werden. Von transformierten Zellen wissen wir, dass sie nachweisbare Mengen Katalase an der Oberfläche tragen, die jedoch in geringerer lokaler Konzentration als bei Tumorzellen vorliegt und daher nicht zum Schutz vor ROS Signaling ausreichend ist. Die ausbleibende Reaktion bei den Normalzellen belegt, dass die Single-Domain-VHH-Fragmente nicht in die Zelle eindringen und die dort vorliegende Katalase beeinflussen können. Sie wirken also spezifisch auf die membranständige Katalase, wie sie vor allem für Tumorzellen charakteristisch ist. Diese sehr wichtige Aussage über den Reaktionsort der Single-Domain-VHH-Fragmente hat aber nur dann Signifikanz, wenn gleichzeitig nachgewiesen werden kann, dass eine Inhibition der intrazellulären Katalase von Normalzellen tatsächlich einen Einfluss auf deren Sensitivität gegen H₂O₂ gehabt hätte.

Dieser Kontrollaspekt wird in Fig. 27 behandelt.

Fig. 27 zeigt, dass das siRNA-vermittelte Ausschalten der intrazellulären Katalase von Normalzellen deren Sensitivität gegen H₂O₂ erhöht.

Normale Zellen (208F), transformierte Zellen (208Fsrc3) und Tumorzellen (MKN-45) wurden mit Kontroll siRNA (siCo) und siRNA gegen Katalase (siCAT) transfiziert und für 24 Stunden bei 37 °C und 5 % CO₂ gehalten. Danach wurden die Zellen in frischem Medium aufgenommen und in einer Zelldichte von 6000 Zellen/100 µl Medium aufgenommen. Die Ansätze wurden anschließend entweder mit steigenden Konzentrationen GOX (A-C) oder Peroxynitrit (PON) (D-F) behandelt. Nach 1.5 Stunden Inkubation bei 37 °C und 5 % CO₂ wurden die Prozentsätze apoptotischer Zellen bestimmt. Zur Bewertung dieses Experimentes ist es notwendig, zu rekapitulieren, dass GOX H₂O₂ generiert, welches sehr gut zellgängig ist und daher sowohl von membranständiger, als auch intrazellulärer Katalase abgebaut werden kann. Exogen zugegebenes Peroxynitrit reagiert hingegen mit der Zellmembran, wenn es auf die Zelle trifft. Ein Schutz vor der Wirkung von Peroxynitrit kann daher nur durch außen an der Membran sitzende Katalase erzielt werden.

Fig. 27 bestätigt zunächst, dass Tumorzellen wesentlich stärker gegen H₂O₂ und Peroxynitrit geschützt sind als normale oder transformierte Zellen. Die Figur zeigt weiterhin, dass ein siRNA-vermittelter Abbau von Katalase eine sehr starke Sensitivierung von Normalzellen, transformierten Zellen und Tumorzellen gegen die Wirkung von H₂O₂ mit sich bringt. Nach Behandlung mit Peroxynitrit zeigte sich ein vollständig anderes Bild: jetzt führt der siRNA-vermittelte Abbau von Katalase in den Normalzellen zu keiner Sensitivierung, während sich bei den transformierten Zellen und Tumorzellen eine starke Sensitivierung einstellt. Damit ist gezeigt, dass sich nur an der Oberfläche der malignen Zellen, also der transformierten und der Tumorzellen, Katalase befindet, die vor exogenem Peroxynitrit schützt. Eine protektive membranständige Katalasewirkung ist bei den Normalzellen nicht nachweisbar. Der mithilfe von H₂O₂-generierenderer GOX erzielte Befund zeigt, dass normale Zellen funktionelle protektive Katalase besitzen, deren Wirkung sich z. B. durch ein siRNA-vermitteltes Abschalten nachweisen lässt. Da in dem in Fig. 26 gezeigten Experiment jedoch eine Sensitivierung von Normalzellen gegen H₂O₂ mithilfe von Katalase-neutralisierenden Single-Domain-VHH-Fragmenten nicht nachweisen lässt, ist nachgewiesen, dass diese nicht im Inneren der Zelle wirken konnten.

### Beispiel 15: Die für Single-Domain-VHH-Fragmente gezeigte Wirkung in vitro korreliert mit einer Hemmung des Tumorwachstums in vivo.

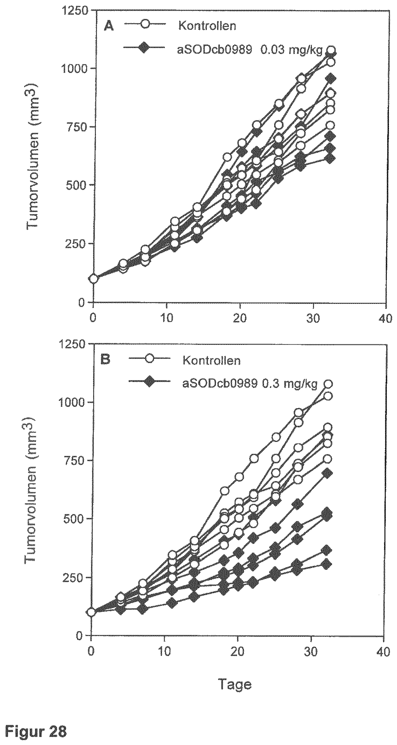

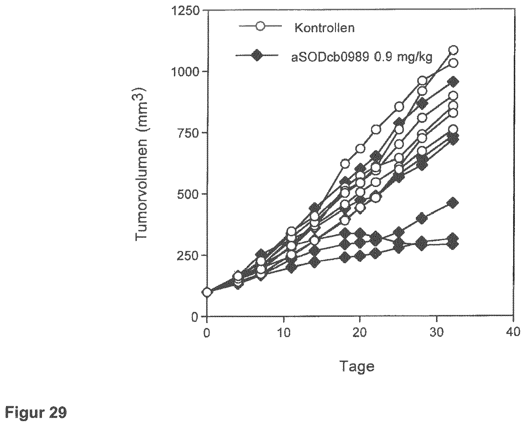

Fig. 28 und 29 zeigen, dass das Wachstum eines humanen Kolonkarzinom-Xenotransplantats auf immunkomprimierten Mäusen durch wiederholte Gabe des Single-Domain-VHH-Fragments aSODcb0989 gehemmt wird.

Die in Fig. 28 und 29 durchgeführten Experimente wurden von einem kommerziellen, zertifizierten Anbieter (Oncotest GmbH, Freiburg) durchgeführt. Dabei wurden Xenotransplantate eines humanen Kolonkarzinoms geeigneten Mäusen implantiert. Nach Anwachsen der Tumoren und nach Erreichen einer bestimmten Mindestgröße wurde der Therapieversuch begonnen. aSODcb0989 in den angezeigten Dosen (mg/kg Körpergewicht) oder Puffer wurde zweimal wöchentlich intravenös appliziert. Die Tumorgrößen wurden ebenfalls zweimal wöchentlich mithilfe eines Kalibers gemessen.

Fig. 28 zeigt zunächst, dass das Tumorwachstum in den Kontrolltieren durch eine sehr deutliche Streuung charakterisiert ist. Bei Applikation von 0.03 mg/kg aSODcb0989 ergibt sich kein erkennbarer Unterschied zu den Kontrollen (Fig. 28 A). Bei Gabe von 0.3 mg/kg aSODcb0989 unterscheidet sich die mit dem Single-Domain-VHH-Fragment behandelte Gruppe sehr deutlich von der Kontrollgruppe, obwohl beide Gruppen einer starken Streuung unterliegen. Der dargestellte Unterschied im Tumorvolumen zwischen Kontrollgruppe und behandelter Gruppe ist der Ausdruck einer deutlichen Proliferationshemmung durch aSODcb0989. Bei weiterer Steigerung der Dosis von aSODcb0989 auf 0.9 mg/kg (Fig. 29), wird bei einigen Tieren die Wachstumshemmung verstärkt, während sie bei anderen Tieren das Gegenteil bewirkt. Dies lässt erkennen, dass auch in vivo eine Dosiswirkungskurve in Form eines Optimums vorliegt und die Bedingungen in dem in Fig. 29 gezeigten Experiment den Grenzbereich der Konzentration zur supraoptimalen Hemmung erreicht haben.
## Patentansprüche

1. Antigen bindendes Konstrukt zur Verwendung bei der Behandlung von Tumorerkrankungen, wobei das Konstrukt spezifisch an humane Superoxiddismutase bindet und diese inhibiert oder an humane Katalase bindet und diese inhibiert, **dadurch gekennzeichnet, dass** es ein Single-Domain-VHH-Fragment ist, und das wenigstens drei CDR Sequenz(en) beinhaltet, wobei die genannten drei CDR sequenzen jeweils aus (a) SEQ ID NO:19 als CDR1, SEQ ID NO:20 als CDR2 und SEQ ID NO:21 als CDR3, oder (b) SEQ ID NO:25 als CDR1, SEQ ID NO:26 als CDR2 und SEQ ID NO:27 als CDR3 oder (c) SEQ ID NO:34 als CDR1, SEQ ID NO:35 als CDR2 und SEQ ID NO:36 als CDR3 ausgewählt sind

2. Antigen bindendes Konstrukt zur Verwendung nach Anspruch 1, **dadurch gekennzeichnet, dass** dieses weiterhin einen Marker aufweist.

3. Antigen bindendes Konstrukt zur Verwendung nach einem der Ansprüche 1 bis 2, **dadurch gekennzeichnet, dass** es mit einem zytotoxischen Mittel verbunden ist.

4. Antigen bindendes Konstrukt zur Verwendung nach einem der Ansprüche 1 bis 3, **dadurch gekennzeichnet, dass** es mit einer Komponente verbunden ist, die die Verweildauer des Konstruktes im Zielorganismus verändert.

5. Pharmazeutische Zusammensetzung zur Verwendung bei der Behandlung von Tumorerkrankungen, **dadurch gekennzeichnet, dass** sie wenigstens ein Antigen bindendes Konstrukt gemäß einem der Ansprüche 1 bis 4 beinhaltet.

6. Pharmazeutische Zusammensetzung zur Verwendung bei der Behandlung von Tumorerkrankungen nach Anspruch 5, **dadurch gekennzeichnet, dass** das Antigen bindende Konstrukt spezifisch an die Katalase bindet und diese inhibiert.

7. Pharmazeutische Zusammensetzung zur Verwendung bei der Behandlung von Tumorerkrankungen nach Anspruch 5, **dadurch gekennzeichnet, dass** das Antigen bindende Konstrukt spezifisch an die Superoxiddismutase bindet und diese inhibiert.

8. Pharmazeutische Zusammensetzung zur Verwendung bei der Behandlung von Tumorerkrankungen nach Anspruch 5, **dadurch gekennzeichnet, dass** sie wenigstens ein Antigen bindendes Konstrukt, das an die Katalase bindet und diese inhibiert, sowie wenigstens ein Konstrukt das spezifisch an die Superoxiddismutase bindet und diese inhibiert, beinhaltet.

9. Pharmazeutische Zusammensetzung zur Verwendung bei der Behandlung von Tumorerkrankungen nach einem der Ansprüche 5 bis 8, **dadurch gekennzeichnet, dass** sie weiterhin ein Tumortherapeutikum, insbesondere Taxol, beinhaltet.

## Claims

1. Antigen-binding construct for use in the treatment of tumour diseases, whereby the construct specifically binds to and inhibits human superoxide dismutase or binds to and inhibits human catalase, **characterized by** being a single-domain-VHH-fragment, comprising at least three CDR sequences; whereby the said three CDR sequences each are selected from
(a) SEQ ID NO:19 as CDR1, SEQ ID NO:20 as CDR2 and SEQ ID NO:21 as CDR3; or
(b) SEQ ID NO:25 as CDR1, SEQ ID NO:26 as CDR2 and SEQ ID NO:27 as CDR3; or
(c) SEQ ID NO:34 as CDR1, SEQ ID NO:35 as CDR2 and SEQ ID NO:36 as CDR3.

2. Antigen-binding construct for use according to claim 1, **characterized by** further exhibiting a marker.

3. Antigen-binding construct for use according to one of the claims 1 to 2, **characterized by** being combined with a cytotoxic agent.

4. Antigen-binding construct for use according to one of the claims 1 to 3, **characterized by** being combined with a component, which alters the retention time of the construct in the target organism.

5. Pharmaceutical composition for use in the treatment of tumour diseases, **characterized by** comprising at least one antigen-binding construct according to one of the claims 1 to 4.

6. Pharmaceutical composition for use in the treatment of tumour diseases according to claim 5, **characterized by** the antigen-binding construct that specifically binds to and inhibits the catalase.

7. Pharmaceutical composition for use in the treatment of tumour diseases according to claim 5, **characterized by** the antigen-binding construct that specifically binds to and inhibits the superoxide dismutase.

8. Pharmaceutical composition for use in the treatment of tumour diseases according to claim 5, **characterized by** comprising at least one antigen-binding construct that, binds to and inhibits the catalase, as well as at least one construct, that specifically binds to and inhibits the superoxide dismutase.

9. Pharmaceutical composition for use in the treatment of tumour diseases according to one of the claims 5 to 8, **characterised by** further comprising a tumour therapeutic agent, in particular, Taxol.

## Revendications

1. Construction se liant à l'antigène pour une utilisation dans le traitement de tumeurs, dans laquelle la construction se lie spécifiquement à la superoxyde dismutase humaine et l'inhibe ou se lie à la catalase humaine et l'inhibe, **caractérisée en ce qu'**elle est un fragment de VHH à domaine unique, et qui contient au moins trois séquences CDR, dans laquelle lesdites trois séquences CDR sont sélectionnées respectivement à partir de (a) SEQ ID N° : 19 en tant que CDR1, SEQ ID N° : 20 en tant que CDR2 et SEQ ID N° : 21 en tant que CDR3, ou (b) SEQ ID N° : 25 en tant que CDR1, SEQ ID N° : 26 en tant que CDR2 et SEQ ID N° : 27 en tant que CDR3 ou (c) SEQ ID N° : 34 en tant que CDR1, SEQ ID N° : 35 en tant que CDR2 et SEQ ID N° : 36 en tant que CDR3

2. Construction se liant à l'antigène pour une utilisation selon la revendication 1, **caractérisée en ce qu'**elle présente en outre un marqueur.

3. Construction se liant à l'antigène pour une utilisation selon l'une des revendications 1 à 2, **caractérisée en ce qu'**elle est reliée à un agent cytotoxique.

4. Construction se liant à l'antigène pour une utilisation selon l'une des revendications 1 à 3, **caractérisée en ce qu'**elle est reliée à un constituant qui modifie la durée de séjour de la construction dans l'organisme cible.

5. Composition pharmaceutique pour une utilisation dans le traitement de tumeurs, **caractérisée en ce qu'**elle contient au moins une construction se liant à l'antigène selon l'une des revendications 1 à 4.

6. Composition pharmaceutique pour une utilisation dans le traitement de tumeurs selon la revendication 5, **caractérisée en ce que** la construction se liant à l'antigène se lie spécifiquement à la catalase et l'inhibe.

7. Composition pharmaceutique pour une utilisation dans le traitement de tumeurs selon la revendication 5, **caractérisée en ce que** la construction se liant à l'antigène se lie spécifiquement à la superoxyde dismutase et l'inhibe.

8. Composition pharmaceutique pour une utilisation dans le traitement de tumeurs selon la revendication 5, **caractérisée en ce qu'**elle contient au moins une construction se liant à l'antigène, qui se lie à la catalase et l'inhibe, ainsi qu'au moins une construction qui se lie spécifiquement à la superoxyde dismutase et l'inhibe.

9. Composition pharmaceutique pour une utilisation dans le traitement de tumeurs selon l'une des revendications 5 à 8, **caractérisée en ce qu'**elle contient en outre un agent thérapeutique anti-tumoral, en particulier le taxol.


<110> Bauer, Georg Motz, Manfred
<120> Antigen bindende Konstrukte
<130> PWO00251BAU
<160> 51
<170> PatentIn version 3.5
<210> 1
   <211> 19
   <212> RNA
   <213> künstliche Sequenz
<220>
   <223> Kontroll siRNA
<400> 1
   uucuccgaac gugucacgu 19
<210> 2
   <211> 19
   <212> RNA
   <213> künstliche Sequenz
<220>
   <223> Kontroll siRNA Antisense
<400> 2
   acgugacacg uucggagaa 19
<210> 3
   <211> 21
   <212> DNA
   <213> künstliche Sequenz
<220>
   <223> si RNA fasr
<400> 3
   aaggagtaca cagacaaagc c 21
<210> 4
   <211> 21
   <212> DNA
   <213> künstliche Sequenz
<220>
   <223> siRNA casp 8
<400> 4
   aagagtctgt gcccaaatca a 21
<210> 5
   <211> 21
   <212> DNA
   <213> künstliche Sequenz
<220>
   <223> siRNA casp 9
<400> 5
   cagtgacatc tttgtgtcct a 21
<210> 6
   <211> 21
   <212> DNA
   <213> künstliche Sequenz
<220>
   <223> siRNA NOX
<400> 6
   ccgacaaata ctactacaca a 21
<210> 7
   <211> 21
   <212> DNA
   <213> künstliche Sequenz
<220>
   <223> siRNA NOS2
<400> 7
   ctgggccgtg caaaccttca a 21
<210> 8
   <211> 21
   <212> DNA
   <213> künstliche Sequenz
<220>
   <223> siRNA cat
<400> 8
   ccggatctca cttggcggca a 21
<210> 9
   <211> 21
   <212> DNA
   <213> künstliche Sequenz
<220>
   <223> siRNA Maus cat
<400> 9
   cccaatagga gataaactta a 21
<210> 10
   <211> 128
   <212> PRT
   <213> künstliches Konstrukt
<220>
   <223> Katalase bindend
<400> 10
   Met Ala Gln Val Gln Leu Val Glu Ser Gly Gly Gly Leu Val Gln Ala
   1               5                   10                  15
   Gly Gly Ser Leu Arg Leu Ser Cys Ala Ala Ser Glu Arg Thr Phe Asn
               20                  25                  30
   Thr Tyr Gly Met Gly Trp Phe Arg Gln Ala Pro Gly Lys Glu Arg Glu
           35                  40                  45
   Phe Val Ala Thr Ile Ser Trp Ser Gly Asp Ser Thr Tyr Tyr Ala Asp
       50                  55                  60
   Ser Val Lys Gly Arg Phe Thr Ile Ser Arg Asp Asn Ala Lys Asn Thr
   65                  70                  75                  80
   Met Tyr Leu Gln Met Asn Ser Leu Lys Pro Glu Asp Thr Ala Val Tyr
                   85                  90                  95
   Tyr Cys Asn Ala Asn Ser Glu Tyr Gly Asp Ser Tyr Trp Gly Gln Gly
               100                 105                 110
   Thr Gln Val Thr Val Ser Ser Lys Lys Lys His His His His His His
           115                 120                 125
<210> 11
   <211> 128
   <212> PRT
   <213> künstliche Sequenz
<220>
   <223> Katalase bindend
<400> 11
   Met Ala Glu Val Gln Leu Val Glu Ser Gly Gly Gly Leu Val Gln Pro
   1               5                   10                  15
   Gly Gly Ser Leu Arg Leu Ser Cys Ala Val Ser Gly Phe Ile Phe Asn
               20                  25                  30
   Thr Tyr Ser Met Arg Trp Gly Arg Gln Ala Pro Gly Lys Gly Leu Glu
           35                  40                  45
   Trp Val Ser Ser Ile Ser Thr Gly Gly Tyr Ser Thr Tyr Ala Asp Ser
       50                  55                  60
   Val Lys Gly Arg Phe Thr Ile Ser Arg Asp Asn Ala Lys Asn Leu Val
   65                  70                  75                  80
   Tyr Leu Gln Met Asn Ser Leu Lys Pro Glu Asp Thr Ala Val Tyr Tyr
                   85                  90                  95
   Cys Gly Trp Gly Ala Phe Val Arg Gly Glu Arg Pro Gln Gly Gln Gly
               100                 105                 110
   Thr Gln Val Thr Val Ser Ser Lys Lys Lys His His His His His His
           115                 120                 125
<210> 12
   <211> 133
   <212> PRT
   <213> künstliche Sequenz
<220>
   <223> Katalase bindend
<400> 12
   Met Ala Gln Val Gln Leu Val Glu Ser Gly Gly Gly Leu Val Gln Pro
   1               5                   10                  15
   Gly Gly Ser Leu Arg Leu Ser Cys Ala Ala Ser Gly Ser Ile Phe Ser
               20                  25                  30
   Ile Ala Ser Met Gly Trp Tyr Arg Gln Ala Pro Gly Lys Gln Arg Asp
           35                  40                  45
   Leu Val Ala Thr Ile Thr Ser Asp Gly Ser Thr Lys Tyr Ala Asp Ser
       50                  55                  60
   Val Lys Gly Arg Phe Thr Ile Ser Arg Asp Asn Ala Lys Asn Thr Met
   65                  70                  75                  80
   Tyr Leu Gln Met Asn Ser Val Lys Pro Glu Asp Ala Ala Val Tyr Tyr
                   85                  90                  95
   Cys Asn Ala Asp Ala Asp Asp Leu Glu Pro Gly Ser Tyr Asp Tyr Asp
               100                 105                 110
   Tyr Trp Gly Gln Gly Thr Gln Val Thr Val Ser Ser Lys Lys Lys His
           115                 120                 125
   His His His His His
       130
<210> 13
   <211> 133
   <212> PRT
   <213> künstliche Sequenz
<220>
   <223> Katalase bindend
<400> 13
   Met Ala Gln Val Gln Leu Val Glu Ser Gly Gly Gly Leu Val Gln Pro
   1               5                   10                  15
   Gly Gly Ser Leu Arg Leu Ser Cys Ala Ala Ser Ala Ser Ile Phe Ser
               20                  25                  30
   Ile Tyr Val Met Ala Trp Tyr Arg Gln Ala Pro Gly Lys Gln Arg Glu
           35                  40                  45
   Leu Val Ala Thr Val Thr Ser Gly Gly Ala Thr Asn Tyr Ala Asn Ser
       50                  55                  60
   Val Lys Gly Arg Phe Thr Ile Ser Arg Asp Asn Ala Lys Asn Thr Met
   65                  70                  75                  80
   Asp Leu Gln Met Asn Ser Leu Lys Pro Glu Asp Thr Ala Val Tyr Tyr
                   85                  90                  95
   Cys Asn Ala Glu Asp Tyr Tyr Asp Tyr Gly Leu Ser Arg Ser Lys Ile
               100                 105                 110
   Tyr Trp Gly Gln Gly Thr Gln Val Thr Val Ser Ser Lys Lys Lys His
           115                 120                 125
   His His His His His
       130
<210> 14
   <211> 127
   <212> PRT
   <213> künstliche Sequenz
<220>
   <223> SOD bindend
<400> 14
   Met Ala Gln Val Gln Leu Val Glu Ser Gly Gly Gly Ile Val Gln Pro
   1               5                   10                  15
   Gly Gly Ser Leu Arg Leu Ser Cys Val Ala Ser Glu Ser Ile Ser Glu
               20                  25                  30
   Ile Asp Ala Met Tyr Trp His Arg Gln Ala Pro Gly Lys Glu Arg Glu
           35                  40                  45
   Leu Val Ala Gly Ile Thr Asn Asp Gly Thr Arg Tyr Tyr Ala Asp Ser
       50                  55                  60
   Val Lys Gly Arg Phe Thr Ile Ser Arg Asp Asn Ala Lys Ser Thr Leu
   65                  70                  75                  80
   Tyr Leu Gln Met Asn Ser Leu Lys Phe Glu Asp Thr Ala Met Tyr Tyr
                   85                  90                  95
   Cys Ala Ala Leu Pro Asn Pro Pro Pro Gly Tyr Trp Gly Gln Gly Thr
               100                 105                 110
   Gln Val Thr Val Ser Ser Lys Lys Lys His His His His His His
           115                 120                 125
<210> 15
   <211> 127
   <212> PRT
   <213> künstliche Sequenz
<220>
   <223> SOD bindend
<400> 15
   Met Ala Gln Val Gln Leu Val Glu Ser Gly Gly Gly Leu Val Gln Ser
   1               5                   10                  15
   Gly Gly Ser Leu Thr Leu Ser Cys Thr Ala Ser Gly Phe Thr Ile Ser
               20                  25                  30
   Asn Tyr Pro Met Thr Trp Val Arg Gln Ala Pro Gly Lys Gly Leu Glu
           35                  40                  45
   Trp Val Ser Arg Ile Asn Ser Gly Gly Asp Arg Thr Leu Tyr Ala Asp
       50                  55                  60
   Ser Val Lys Gly Arg Phe Thr Val Ser Arg Asp Asn Ala Arg Asn Thr
   65                  70                  75                  80
   Met Tyr Leu Gln Met Asn Asn Leu Lys Pro Glu Asp Thr Gly Leu Tyr
                   85                  90                  95
   Phe Cys Ala Asp Ser Gly Ala Gly Trp Arg Tyr Trp Gly Gln Gly Thr
               100                 105                 110
   Gln Val Thr Val Ser Ser Lys Lys Lys His His His His His His
             115                 120                 125
<210> 16
   <211> 127
   <212> PRT
   <213> künstliche Sequenz
<220>
   <223> SOD bindend
<400> 16
   Met Ala Gln Val Gln Leu Val Glu Ser Gly Gly Gly Ile Val Gln Pro
   1               5                   10                  15
   Gly Gly Ser Leu Arg Leu Ser Cys Val Ala Ser Glu Ser Ile Ser Asp
               20                  25                  30
   Ile Asp Ala Met Tyr Trp His Arg Gln Ala Pro Gly Lys Arg Arg Glu
           35                  40                  45
   Leu Val Ala Gly Ile Thr Asn Asp Gly Thr Glu Tyr Phe Ala Asp Ser
       50                  55                  60
   Val Lys Gly Arg Phe Ala Ile Ser Arg Asp Asn Thr Lys Ser Ser Leu
   65                  70                  75                  80
   Tyr Leu Gln Met Asn Ser Leu Lys Leu Glu Asp Thr Ala Met Tyr Tyr
                   85                  90                  95
   Cys Ala Thr Leu Pro Asn Pro Pro Pro Gly Tyr Trp Gly Gln Gly Thr
               100                 105                 110
   Gln Val Thr Val Ser Ser Lys Lys Lys His His His His His His
           115                 120                 125
<210> 17
   <211> 260
   <212> PRT
   <213> künstliche Sequenz
<220>
   <223> bispezifisch
<400> 17
   Met Ala Gln Val Gln Leu Val Glu Ser Gly Gly Gly Leu Val Gln Ala
   1               5                   10                  15
   Gly Gly Ser Leu Arg Leu Ser Cys Ala Ala Ser Glu Arg Thr Phe Asn
               20                  25                  30
   Thr Tyr Gly Met Gly Trp Phe Arg Gln Ala Pro Gly Lys Glu Arg Glu
   35 40 45
   Phe Val Ala Thr Ile Ser Trp Ser Gly Asp Ser Thr Tyr Tyr Ala Asp
   50 55 60
   Ser Val Lys Gly Arg Phe Thr Ile Ser Arg Asp Asn Ala Lys Asn Thr
   65 70 75 80
   Met Tyr Leu Gln Met Asn Ser Leu Lys Pro Glu Asp Thr Ala Val Tyr
   85 90 95
   Tyr Cys Asn Ala Asn Ser Glu Tyr Gly Asp Ser Tyr Trp Gly Gln Gly
   100 105 110
   Thr Gln Val Thr Val Ser Ser Gly Gly Gly Gly Ser Gly Gly Gly Gly
   115 120 125
   Ser Gly Gly Gly Gly Ser Ala Gln Val Gln Leu Val Glu Ser Gly Gly
   130 135 140
   Gly Leu Val Gln Ser Gly Gly Ser Leu Thr Leu Ser Cys Thr Ala Ser
   145 150 155 160
   Gly Phe Thr Ile Ser Asn Tyr Pro Met Thr Trp Val Arg Gln Ala Pro
   165 170 175
   Gly Lys Gly Leu Glu Trp Val Ser Arg Ile Asn Ser Gly Gly Asp Arg
   180 185 190
   Thr Leu Tyr Ala Asp Ser Val Lys Gly Arg Phe Thr Val Ser Arg Asp
   195 200 205
   Asn Ala Arg Asn Thr Met Tyr Leu Gln Met Asn Asn Leu Lys Pro Glu
   210 215 220
   Asp Thr Gly Leu Tyr Phe Cys Ala Asp Ser Gly Ala Gly Trp Arg Tyr
   225 230 235 240
   Trp Gly Gln Gly Thr Gln Val Thr Val Ser Ser Lys Lys Lys His His
   245 250 255
   His His His His
   260
<210> 18
   <211> 260
   <212> PRT
   <213> künstliche Sequenz
<220>
   <223> bispezifisch
<400> 18
   Met Ala Gln Val Gln Leu Val Glu Ser Gly Gly Gly Leu Val Gln Ser
   1               5                   10                  15
   Gly Gly Ser Leu Thr Leu Ser Cys Thr Ala Ser Gly Phe Thr Ile Ser
               20                  25                  30
   Asn Tyr Pro Met Thr Trp Val Arg Gln Ala Pro Gly Lys Gly Leu Glu
           35                  40                  45
   Trp Val Ser Arg Ile Asn Ser Gly Gly Asp Arg Thr Leu Tyr Ala Asp
       50                  55                  60
   Ser Val Lys Gly Arg Phe Thr Val Ser Arg Asp Asn Ala Arg Asn Thr
   65                  70                  75                  80
   Met Tyr Leu Gln Met Asn Asn Leu Lys Pro Glu Asp Thr Gly Leu Tyr
                   85                  90                  95
   Phe Cys Ala Asp Ser Gly Ala Gly Trp Arg Tyr Trp Gly Gln Gly Thr
               100                 105                 110
   Gln Val Thr Val Ser Ser Gly Gly Gly Gly Ser Gly Gly Gly Gly Ser
           115                 120                 125
   Gly Gly Gly Gly Ser Ala Gln Val Gln Leu Val Glu Ser Gly Gly Gly
       130                 135                 140
   Leu Val Gln Ala Gly Gly Ser Leu Arg Leu Ser Cys Ala Ala Ser Glu
   145                 150                 155                 160
   Arg Thr Phe Asn Thr Tyr Gly Met Gly Trp Phe Arg Gln Ala Pro Gly
                   165                 170                 175
   Lys Glu Arg Glu Phe Val Ala Thr Ile Ser Trp Ser Gly Asp Ser Thr
               180                 185                 190
   Tyr Tyr Ala Asp Ser Val Lys Gly Arg Phe Thr Ile Ser Arg Asp Asn
           195                 200                 205
   Ala Lys Asn Thr Met Tyr Leu Gln Met Asn Ser Leu Lys Pro Glu Asp
       210                 215                 220
   Thr Ala Val Tyr Tyr Cys Asn Ala Asn Ser Glu Tyr Gly Asp Ser Tyr
   225 230 235 240
   Trp Gly Gln Gly Thr Gln Val Thr Val Ser Ser Lys Lys Lys His His
   245 250 255
   His His His His
   260
<210> 19
   <211> 9
   <212> PRT
   <213> künstliche Sequenz
<220>
   <223> CDR
<400> 19
   Arg Thr Phe Asn Thr Tyr Gly Met Gly
   1 5
<210> 20
   <211> 17
   <212> PRT
   <213> künstliche Sequenz
<220>
   <223> CDR
<400> 20
   Thr Ile Ser Trp Ser Gly Asp Ser Thr Tyr Tyr Ala Asp Ser Val Lys
   1 5 10 15
   Gly
<210> 21
   <211> 8
   <212> PRT
   <213> künstliche Sequenz
<220>
   <223> CDR
<400> 21
   Asn Ser Glu Tyr Gly Asp Ser Tyr
   1 5
<210> 22
   <211> 9
   <212> PRT
   <213> künstliche Sequenz
<220>
   <223> CDR
<400> 22
   Phe Ile Phe Asn Thr Tyr Ser Met Arg
   1 5
<210> 23
   <211> 16
   <212> PRT
   <213> künstliche Sequenz
<220>
   <223> CDR
<400> 23
   Ser Ile Ser Thr Gly Gly Tyr Ser Thr Tyr Ala Asp Ser Val Lys Gly
   1 5 10 15
<210> 24
   <211> 9
   <212> PRT
   <213> künstliche Sequenz
<220>
   <223> CDR
<400> 24
   Gly Ala Phe Val Arg Gly Glu Arg Pro
   1 5
<210> 25
   <211> 9
   <212> PRT
   <213> künstliche Sequenz
<220>
   <223> CDR
<400> 25
   Ser Ile Phe Ser Ile Ala Ser Met Gly
   1 5
<210> 26
   <211> 16
   <212> PRT
   <213> künstliche Sequenz
<220>
   <223> CDR
<400> 26
   Thr Ile Thr Ser Asp Gly Ser Thr Lys Tyr Ala Asp Ser Val Lys Gly
   1 5 10 15
<210> 27
   <211> 14
   <212> PRT
   <213> künstliche Sequenz
<220>
   <223> CDR
<400> 27
   Asp Ala Asp Asp Leu Glu Pro Gly Ser Tyr Asp Tyr Asp Tyr
   1 5 10
<210> 28
   <211> 9
   <212> PRT
   <213> künstliche Sequenz
<220>
   <223> CDR
<400> 28
   Ser Ile Phe Ser Ile Tyr Val Met Ala
   1 5
<210> 29
   <211> 16
   <212> PRT
   <213> künstliche Sequenz
<220>
   <223> CDR
<400> 29
   Thr Val Thr Ser Gly Gly Ala Thr Asn Tyr Ala Asn Ser Val Lys Gly
   1 5 10 15
<210> 30
   <211> 14
   <212> PRT
   <213> künstliche Sequenz
<220>
   <223> CDR
<400> 30
   Glu Asp Tyr Tyr Asp Tyr Gly Leu Ser Arg Ser Lys Ile Tyr
   1 5 10
<210> 31
   <211> 9
   <212> PRT
   <213> künstliche Sequenz
<220>
   <223> CDR
<400> 31
   Ser Ile Ser Glu Ile Asp Ala Met Tyr
   1               5
<210> 32
   <211> 16
   <212> PRT
   <213> künstliche Sequenz
<220>
   <223> CDR
<400> 32
   Gly Ile Thr Asn Asp Gly Thr Arg Tyr Tyr Ala Asp Ser Val Lys Gly
   1               5                   10                  15
<210> 33
   <211> 8
   <212> PRT
   <213> künstliche Sequenz
<220>
   <223> CDR
<400> 33
   Leu Pro Asn Pro Pro Pro Gly Tyr
   1               5
<210> 34
   <211> 9
   <212> PRT
   <213> künstliche Sequenz
<220>
   <223> CDR
<400> 34
   Phe Thr Ile Ser Asn Tyr Pro Met Thr
   1               5
<210> 35
   <211> 17
   <212> PRT
   <213> künstliche Sequenz
<220>
   <223> CDR
<400> 35
   Arg Ile Asn Ser Gly Gly Asp Arg Thr Leu Tyr Ala Asp Ser Val Lys
   1               5                   10                  15
   Gly
<210> 36
   <211> 7
   <212> PRT
   <213> künstliche Sequenz
<220>
   <223> CDR
<400> 36
   Ser Gly Ala Gly Trp Arg Tyr
   1               5
<210> 37
   <211> 9
   <212> PRT
   <213> künstliche Sequenz
<220>
   <223> CDR
<400> 37
   Ser Ile Ser Asp Ile Asp Ala Met Tyr
   1               5
<210> 38
   <211> 16
   <212> PRT
   <213> künstliche Sequenz
<220>
   <223> CDR
<400> 38
   Gly Ile Thr Asn Asp Gly Thr Glu Tyr Phe Ala Asp Ser Val Lys Gly
   1               5                   10                  15
<210> 39
   <211> 8
   <212> PRT
   <213> künstliche Sequenz
<220>
   <223> CDR
<400> 39
   Leu Pro Asn Pro Pro Pro Gly Tyr
   1 5
<210> 40
   <211> 9
   <212> PRT
   <213> künstliche Sequenz
<220>
   <223> CDR
<400> 40
   Arg Thr Phe Asn Thr Tyr Gly Met Gly
   1 5
<210> 41
   <211> 17
   <212> PRT
   <213> künstliche Sequenz
<220>
   <223> CDR
<400> 41
   Thr Ile Ser Trp Ser Gly Asp Ser Thr Tyr Tyr Ala Asp Ser Val Lys
   1 5 10 15
   Gly
<210> 42
   <211> 8
   <212> PRT
   <213> künstliche Sequenz
<220>
   <223> CDR
<400> 42
   Asn Ser Glu Tyr Gly Asp Ser Tyr
   1 5
<210> 43
   <211> 9
   <212> PRT
   <213> künstliche Sequenz
<220>
   <223> CDR
<400> 43
   Phe Thr Ile Ser Asn Tyr Pro Met Thr
   1 5
<210> 44
   <211> 17
   <212> PRT
   <213> künstliche Sequenz
<220>
   <223> CDR
<400> 44
   Arg Ile Asn Ser Gly Gly Asp Arg Thr Leu Tyr Ala Asp Ser Val Lys
   1               5                   10                  15
   Gly
<210> 45
   <211> 7
   <212> PRT
   <213> künstliche Sequenz
<220>
   <223> CDR
<400> 45
   Ser Gly Ala Gly Trp Arg Tyr
   1               5
<210> 46
   <211> 9
   <212> PRT
   <213> künstliche Sequenz
<220>
   <223> CDR
<400> 46
   Phe Thr Ile Ser Asn Tyr Pro Met Thr
   1               5
<210> 47
   <211> 17
   <212> PRT
   <213> künstliche Sequenz
<220>
   <223> CDR
<400> 47
   Arg Ile Asn Ser Gly Gly Asp Arg Thr Leu Tyr Ala Asp Ser Val Lys
   1               5                   10                  15
   Gly
<210> 48
   <211> 7
   <212> PRT
   <213> künstliche Sequenz
<220>
   <223> CDR
<400> 48
   Ser Gly Ala Gly Trp Arg Tyr
   1               5
<210> 49
   <211> 9
   <212> PRT
   <213> künstliche Sequenz
<220>
   <223> CDR
<400> 49
   Arg Thr Phe Asn Thr Tyr Gly Met Gly
   1               5
<210> 50
   <211> 17
   <212> PRT
   <213> künstliche Sequenz
<220>
   <223> CDR
<400> 50
   Thr Ile Ser Trp Ser Gly Asp Ser Thr Tyr Tyr Ala Asp Ser Val Lys
   1               5                   10                  15
   Gly
<210> 51
   <211> 8
   <212> PRT
   <213> künstliche Sequenz
<220>
   <223> CDR
<400> 51
   Asn Ser Glu Tyr Gly Asp Ser Tyr
   1               5